# EUROPEAN PATENT SPECIFICATION

(11) **EP 0 722 442 B1**
(45) Date of publication and mention of the grant of the patent: **15.01.2003**
(21) Application number: 94927742.0
(22) Date of filing: 29.09.1994
(51) Int. Cl.: C07D 257/02, C07D 401/14, A61K 49/00

(54) **POLYAZACYCLOALKANES AS DICHELANTS**
POLYAZACYCLOALKANE ALS DOPPELTE CHELATBILDNER
POLYAZACYCLOALCANES UTILISES COMME AGENTS DICHELATANTS

(30) Priority: 01.10.1993 GB 9320277
(43) Date of publication of application: 24.07.1996
(73) Proprietor: NYCOMED SALUTAR, INC., Sunnyvale, California 94086 (US)
(72) Inventor: CARVALHO, Joan, Mountain View, CA 94040 (US); FELLMANN, Jere, Douglas, Livermore, CA 94550 (US); WATSON, Alan, David, Campbell, CA 95008 (US); KOO, Michael, San Jose, CA 95134 (US)
(74) Representative: Hammett, Audrey Grace Campbell
(86) International application number: GB9402115
(87) International publication number: WO95009848

(56) References cited:
- EP-A- 0 233 619
- EP-A- 0 255 471
- EP-A- 0 305 320
- EP-A- 0 331 616
- EP-A- 0 485 045
- WO-A-90/12050
- WO-A-91/05762
- WO-A-95/07270
- DATABASE WPI Derwent Publications Ltd., London, GB; AN 87-139178 & JP,A,62 077 374 (SHIMADZU SEISAKUSHO K.K.) , 9 April 1987

## Description

### FIELD OF THE INVENTION

The present invention relates to dichelants, that is chelating agents capable of complexing two metal ions simultaneously, and to chelates and salts thereof and their use in diagnostic and therapeutic compositions, especially as contrast enhancing agents in diagnostic medical imaging.

### BACKGROUND OF THE INVENTION

The medical use of chelants is now well established, for example as stabilisers for pharmaceutical preparations, as antidotes for poisonous heavy metal species, as carriers for diagnostically or therapeutically useful metal ions, for example in contrast media for use in magnetic resonance, X-ray or ultrasound imaging or in scintigraphy.

For such diagnostic agents, it is generally important that the chelate complexes should be stable both kinetically and thermodynamically and for this reason there has been much interest in the macrocyclic polyamine-based chelates, in particular DOTA and its derivatives and analogues, which form very stable complexes with the lanthanide metal ions such as gadolinium and dysprosium which are favoured diagnostic metal ions for magnetic resonance imaging due to their relatively large effects on the relaxation times (e.g. T₁ and T₂*) of neighbouring water protons.

The paramagnetic lanthanide metal ions useful as MR imaging contrast agents are relatively toxic and for clinical use must be administered in a form which allows little or no release of the metal for subsequent biological uptake and retention. For this reason, from the early years of MR contrast agents, the use of stable chelate complexes has been proposed. Thus the first commercial lanthanide based MR imaging contrast agent, Magnevist, contained GdDTPA, a complex with a high stability constant which following parenteral administration is excreted relatively rapidly by glomerular filtration with the gadolinium still in the chelate complex.

GdDOTA has an even higher pK_{ML} and thus was also a prime candidate for consideration as an MR imaging contrast agent. DOTA (1,4,7,10-tetraazacyclododecane-N,N',N",N'''-tetraacetic acid) and HPDO3A (1-(2-hydroxypropyl)-4,7,10-tetraazacyclododecane-N,N',N''-triacetic acid) have indeed been proposed as chelants for MR imaging contrast agents and GdDOTA and GdHPDO3A have been commercially developed by companies active in this field.

The lanthanide metals generally have a stable +3 oxidation state and DOTA with its four carboxylic acid groups results in a charged complex, i.e. GdDOTA⁻, requiring a counterion. Analogous uncharged complexes may be produced by eliminating one of DOTA's nitrogen-attached carboxymethyl groups or by replacing it by a non-ionizing group, i.e. by using a chelant such as DO3A (1,4,7,10-tetraazacyclododecane-N,N',N"-triacetic acid) or HPDO3A.

Contrast media based on such non-ionic, or overall charge neutral, complexes have lower osmolalities for a given metal ion concentration and can demonstrate other improved properties relative to the analogous charged complexes. Moreover, the ring nitrogen "freed" by removal of the carboxymethyl group in moving from DOTA to DO3A can of course be substituted by groups which can act to enhance the hydrophilicity or lipophilicity or other biodistribution affecting properties of the chelate.

Recently, there has been growing interest in the use of chelants capable of chelating more than one metal ion per chelant molecule as carriers for paramagnetic or heavy metal ions for MR or X-ray imaging contrast agents. These polychelants offer several advantages over the monochelants such as DTPA, DO3A or DOTA. Thus for example, the osmolality at a given metal concentration can be reduced still further, the simultaneous delivery of a plurality of metal ions to a target site can be facilitated, and more efficient contrast agents can be produced.

Polychelants range from dichelants through oligochelants to true polychelants having perhaps hundreds of chelant moieties per molecule. Many such compounds have been described but there is still a need for polychelants, and in particular oligochelants and especially dichelants, having improved properties in terms for example of relaxivity, stability, biodistribution, biotolerability, viscosity, solubility and osmolality.

Particular macrocyclic dichelants described in the literature include the DO3A dimers of formula I, II, III and IV whose preparation has been described by Nycomed Salutar in WO-A-91/05762 and Schering AG in EP-A-255471 and EP-A-485045 (US-A-5277895) and elsewhere. 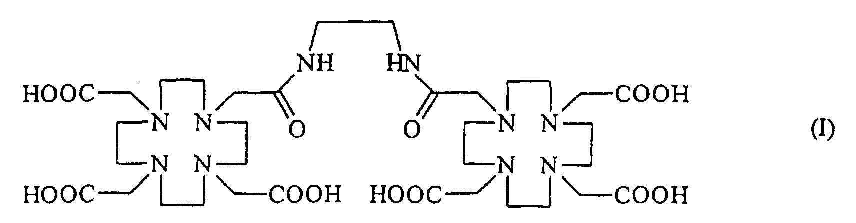 (described by Nycomed Salutar in WO-A-91/05762 and USSN 07/855028) 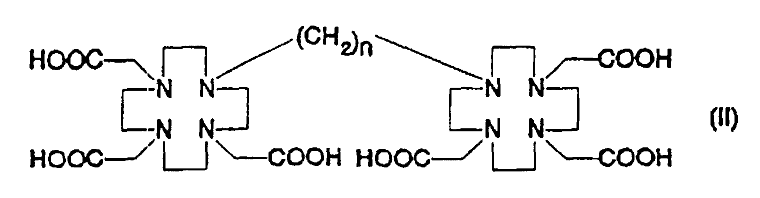 **(where n = 2, 3, 5 or 6)**
(described by Schering AG in EP-A-255471 and in a poster presented at the European Congress of NMR in Medicine and Biology at Strasbourg in May 1990) 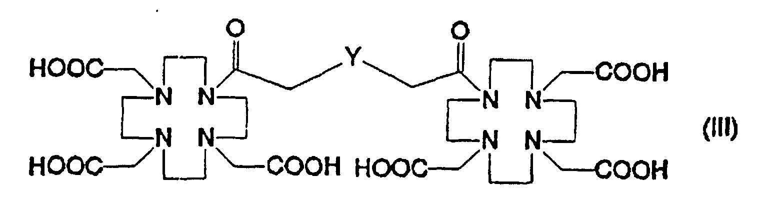 **(where Y is [-N(CH**_{**2**}**COOH)CH**_{**2**}**CH**_{**2**}**N(CH**_{**2**}**COOH)CH**_{**2**}**CH**_{**2**}**N(CH**_{**2**}**COOH)**^{**-**}**]**_{**a**} **and a = 0 or 1)**
(described by Schering AG in EP-A-255471) 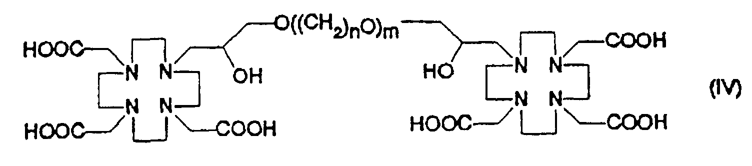 **(where n = 2 or 4 and m = 0 or 1)**
(described by Schering AG in EP-A-485045 (US-A-5277895).

All of these macrocyclic chelant dimers have the general formula DO3A'-L-D03A' where DO3A' is a ring nitrogen deprotonated DO3A residue and L is a linker group.

With lanthanides such as gadolinium, these macrocyclic dimers will produce non-ionic dichelates and these compounds have been found to possess high relaxivity. Thus for example the T1 relaxivities of the bisgadolinium chelates of the compounds of formula II are almost double the T1 relaxivity of GdD03A.

### SUMMARY OF THE INVENTION

We have now found that dichelant compounds having improved properties are produced if the linker groups incorporate ester or amide functionalities, and especially where the linkers comprise carbonyl-attached alkylene groups in which two or more of the methylene groups are replaced by nitrogen or oxygen atoms.

Thus viewed from one aspect the invention provides a polychelant of formula V 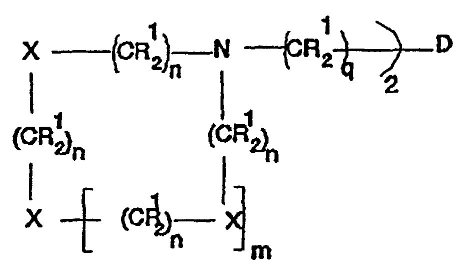 and salts or metal chelates thereof,
(wherein each X which may be the same or different is NZ, O or S, at least two Xs being NZ;
each Z is a group R¹ or a group CR¹₂Y, at least one Z on each macrocyclic ring being a group CR¹₂Y;
each Y is a group CO₂H, PO₃H, SO₃H, CONR¹₂, CON(OR¹)R¹, CNS or CONR¹NR¹₂;
m is 0 or 1 or 2;
each n is 2 or 3;
q is 1 or 2;
each R¹ which may be the same or different is a hydrogen atom or a C₁₋₆ alkyl group optionally substituted by one or more hydroxy and/or C₁₋₆ alkoxy groups;
and D is a bridging group, other than an unsubstituted carbonylaminoethylaminocarbonyl group, of formula

-CO-X²-L¹ (̵X²-CO )̵ₚ

having a molecular weight of less than 1000,
containing one or more ether oxygens,
where p is 0 or 1,
X2 is 0 or NR²,
R² is a hydrogen atom or a hydroxy, OR¹ or NR¹₂ group or a C₁₋₆ alkyl group optionally interrupted by oxygen, sulphur or nitrogen atoms or by carbonyl or aryl groups and optionally substituted by hydroxyl, amine or aryl groups, or R² contains a functional group for attachment to a biomolecule or macromolecule, or two R² groups together form a bridging linker group, and L¹ which provides a chain or at least two atoms linking two X² groups or at least one atom linking an X² group and a (CR¹₂)_{q} moiety (wherein R¹ and q are as defined above), is a straight chain, branched or cyclic alkylene group or a combination of such groups interrupted by oxygen, optionally substituted and optionally being interrupted by sulphur or nitrogen atoms or by aryl or carbonyl groups.

Viewed from another aspect, the present invention provides a detoxification agent comprising a chelating agent according to the invention in the form of a weak complex or salt with a physiologically acceptable counterion, together with at least one pharmaceutical or veterinary carrier or excipient, or adapted for formulation therewith or for inclusion in a pharmaceutical formulation for human or veterinary use.

Viewed from a further aspect, the present invention provides a diagnostic or therapeutic agent comprising a metal chelate, whereof the chelating entity is the residue of a compound according to the present invention, together with at least one pharmaceutical or veterinary carrier or excipient, or adapted for formulation therewith or for inclusion in a pharmaceutical formulation for human or veterinary use.

Viewed from a still further aspect, the present invention provides a process for the preparation of the metal chelates of the invention which process comprises admixing in a solvent a compound of formula V or a salt (e.g. the sodium salt) or chelate thereof together with an at least sparingly soluble compound of said metal, for example a chloride, oxide, acetate or carbonate.

### DETAILED DESCRIPTION

In the compounds of the invention, the macrocyclic 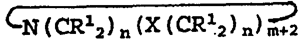 rings preferably have 9 to 14 ring atoms, the ring heteroatoms especially preferably being either all nitrogen or being one oxygen and three nitrogens. The alkylene ring segments (CR¹₂)ₙ preferably are all (CR¹₂)₂ groups or in the case of an N₄ macrocycle the alkylene segments may alternatively be alternating (CR¹₂)₃ and (CR¹₂)₂ groups. Thus the preferred macrocyclic skeletons are those of formulae VI

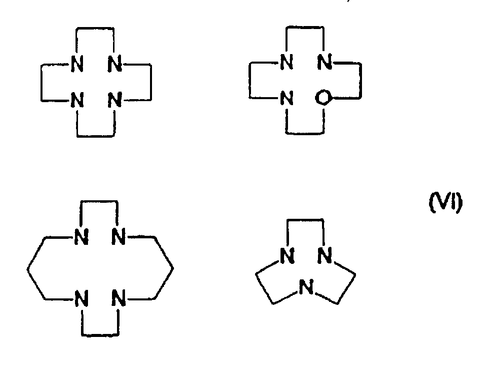

The bridging group D is conveniently a group of formula

-CO-X²-L¹ (̵X²-CO )̵ₚ

where p is 0 or 1,
X² is O or NR²,
R² is a hydrogen atom or a hydroxy, OR¹ or NR¹₂ group or an alkyl group optionally interrupted by oxygen, sulphur or nitrogen atoms or by carbonyl or aryl groups and optionally substituted by hydroxyl, amine or aryl groups, or R² contains a functional group for attachment to a biomolecule or macromolecule, or two R² groups together form a bridging linker group, e.g. a group L¹, and L¹ which provides a chain of at least two atoms linking two X² groups or at least one atom linking an X² group and a (CR¹₂)_{q} moiety (wherein R¹ and q are as hereinbefore defined), is a straight chain, branched or cyclic alkylene group or a combination of such groups, optionally substituted and optionally being interrupted by oxygen, sulphur or nitrogen atoms or by aryl or carbonyl groups.

The linker group L¹ will preferably be a linear, branched or cyclic alkylene group or a combination thereof or a combination of arylene and alkylene groups, for example providing a linking backbone 1 to 50 atoms long but preferably 2 to 25, and especially 2 to 10 atoms long in total on any one unbranched segment. The carbon backbone in such linker groups may be interrupted by heteroatoms such as nitrogen, oxygen and sulphur, and may carry bridging groups, thereby creating homo- or heterocyclic rings within the linker group. Where this occurs, the rings created will preferably be 3 to 12, especially 5 to 8 and, particularly, 6 membered rings. Moreover the rings and the linear segments of the linker group may optionally be unsaturated and may optionally carry one or more substituents selected from alkyl, hydroxy, alkoxy, amine, aryl and substituted aryl groups as well as non-hydrogen R¹ groups or additional chelating groups, eg. Y groups especially carbonyl and SO₃H groups, and groups such as for example long chain (eg. C₁₀₋₂₀) alkyl, aryl or polyaryl groups which are suitable for liposomal incorporation of the compound of formula V or groups, such as isothiocyanate groups, for attachment of the compound of formula V to a biomolecule, polymer, dendrimer or other macromolecule, for example to create a bifunctional chelant.

The bridging group D in the compounds of the invention may, as indicated above, serve to link together two chelant moieties, thereby holding together the dichelate structure. Besides filling this role as linker or spacer of chelant sites, the bridging group can be so selected as to yield a product having other desired characteristics. For example it is possible to increase hydrophilicity, lipophilicity, or tissue specificity of the end product by attaching to or incorporating within the bridging group, groups which are hydrophilic, lipophilic, or tissue targeting. In this way, the overall charge of the chelate structure, or the overall lipophilicity, or tissue targeting can be controlled.

In the compounds of formula V, alkyl moieties preferably have 1 to 6, especially 1 to 4 carbon atoms unless otherwise specified, and aryl moieties are preferably phenyl groups.

Particularly preferred compunds of formula V include those of formula VII

M-CH₂CO)₂-D' (VII)

where M is a nitrogen attached triaza, tetraaza, triazaoxa or triazathia-cycloalkane of formula VI having at least one and preferably two ring nitrogens substituted by CH₂COOH groups and having any remaining ring nitrogen substituted by a group R³, M preferably being a group of formula VI having two or more, preferably three, ring heteroatoms substitued by CH₂COOH groups; R³ (which for tetraheteroatom rings is preferably at the ring heteroatom remote from the ring attachment nitrogen) is a hydrogen atom, or an alkyl group optionally mono or polysubstituted by hydroxyl or alkoxy groups (eg. hydroxyalkyl, polyhydroxyalkyl, alkoxyalkyl, polyalkoxyalkyl, hydroxyalkoxyalkyl, hydroxypolyalkoxyalkyl, polyethylene glycol etc.) and optionally interrupted by arylene or substituted arylene groups; and CO-D'-CO is a bridging group D as discussed above.

Preferred bridging groups D in the dichelant compounds of the invention include those of the formulae: 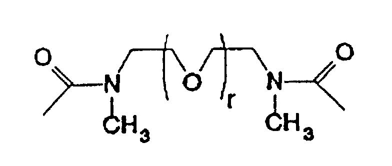 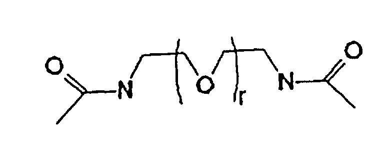 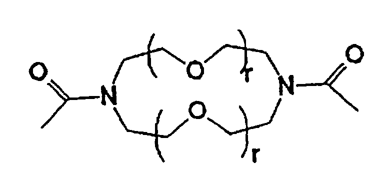 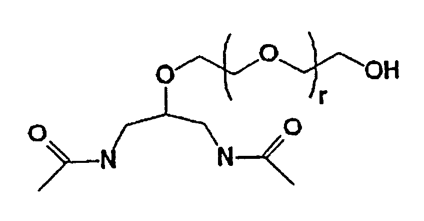 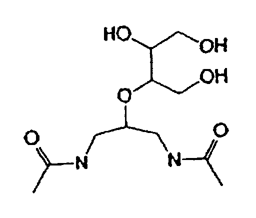 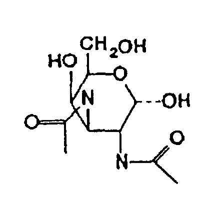 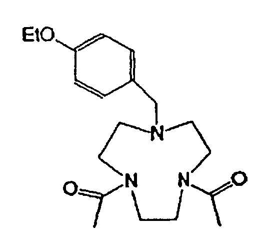 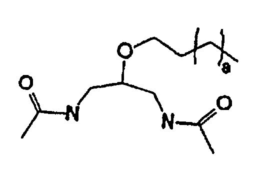 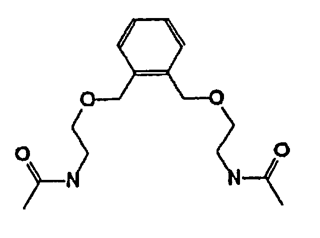 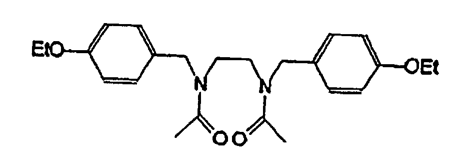 where r is an integer having a value of 1 to 6, especially 1, 2 or 3, and s is an integer having a value of 1 to 20, especially 1 to 15.

The bridging groups D are groups containing ether oxygens, e.g. COOCH₂CH₂OCH₂CH₂NHCH₂CH₂OCH₂CH₂OCO, as these may be associated with particularly low toxicity. Also especially preferred are bridging groups containing amide linkages, for example CONHCH₂CH₂OCH₂CH₂OCH₂CH₂NHCO.

The D groups can, as seen above, carry substituent side chains and where D is a carbonylaminoethylaminocarbonyl group, it will be so substituted, said substitution including the required ether. Example substituent groups include optionally hydroxylated, optionally carboxylated alkyl or oxaalkyl groups optionally interrupted by carbonyamino or arylene functions.

Preferred compounds according to the invention also include the polyhydroxylated dichelants, e.g. where D or R¹ groups are hydroxylated, as they can present a better toxicity profile than their non-hydroxylated counterparts. Lipophilic analogs are also of interest due to the potential for increased liver uptake; moreover the long chain lipophilic analogs have the ability to be incorporated into liposomes for use as blood pool agents and for targetted delivery to specific organs or tissues. Indeed chelate compounds according to the invention, in particular the lipophilic analogs, are of particular interest as blood pool agents where they exhibit prolonged plasma half lifes.

The compounds of the invention may be prepared by conventional synthetic techniques, conveniently starting from the corresponding N-unsubstituted or N-carboxymethylated polyazacycloalkanes, condensing these to a bifunctional linker molecule, generally after protection of one or more of the ring nitrogens or N-carboxymethyl groups, followed by deprotection and if required introduction of functional groups at the ring nitrogens.

Where the dichelant is asymmetrical, it can of course be constructed by conjugating one macrocycle to a monoprotected bifunctional linker molecule, deprotecting, and conjugating a second macrocycle to the macrocycle-linker intermediate. For such purposes, the bifunctional linker molecule may of course itself be asymmetric to allow one end to conjugate directly to a macrocycle ring nitrogen and the other to conjugate to a macrocycle side chain, e.g. a carboxymethyl group or a reactive derivative thereof.

Thus viewed from a further aspect the invention also provides a process for the preparation of the compounds of the invention, said process comprising at least one of the following steps:
(a) reacting a compound of formula V wherein at least one group X is a group NH, with a compound of formula VIII

   Lv-R⁴ (VIII)

   (where Lv is a displaceable leaving group, for example a halogen atom or a substituted sulphonyloxy group, such as chlorine, bromine, iodine, methanesulphonyloxy, phenysulphonyloxy or p-toluenesulphonyloxy groups) and R⁴ is a group CR¹₂Y or group R¹ other than hydrogen);
(b) reacting compounds of formulae X and/or XI 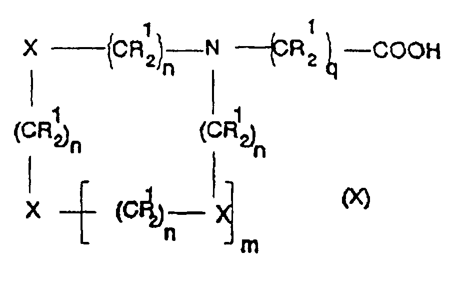 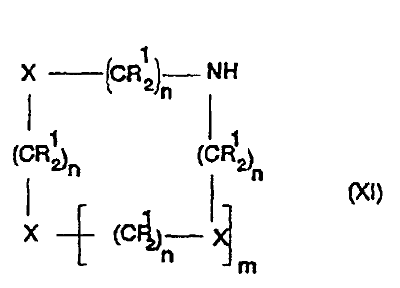 (wherein X, R¹, n, q and m are as hereinbefore defined) or an activated derivative, e.g. halide, thereof with a linker molecule of formula IX

   Lv(CO-X² )̵L¹-(X²-CO)ₜLv (IX)

   or formula IXa

   Lv-(CR¹ ₂)_{q}-(CO-X² )̵L¹-(X²-CO)ₜ-(CR¹ ₂)_{q}-Lv (IXa)

   (wherein X², L¹, R¹ and q are as hereinbefore defined, t is 0 or 1, and each Lv is a displaceable leaving group, one optionally being protected prior to conjugation of the second compound of formula X or XI);
(c) converting a compound of formula X into a corresponding acid chloride and reacting with a suitable diamine;
(d) metallating or transmetallating a compound of formula V or a chelate thereof;
(e) converting a compound of formula V or a chelate thereof into a base or acid addition salt thereof or converting a salt into the free acid or base; and
(f) performing at least one of steps (a) to (d) above using reagents with protected functional groups and subsequently removing the protecting groups.

The starting compounds of formulae VIII, IX, X and XI are either known from the literature or can be produced by conventional synthetic techniques. The starting compounds of formula VIII used in step (a) can be prepared by the process of step (b).

As indicated above, during the reaction, functional groups present in the starting materials but not involved in the particular process steps may be protected, for example to avoid unwanted substitution or polymerisation. Conventional protection and deprotection techniques may be used (see for example "Protective Groups in Organic Synthesis" by T. W. Greene, Wiley-Interscience, NY, 1981 and "Protective Groups in Organic Chemistry" by JFW McOmie, Plenum, London, 1973). Suitable protecting groups for carboxyl groups include ester functions, for ring nitrogens alkyl, borane or organometallic functions, for hydroxyl groups acyl functions. The protecting groups will be removed by standard techniques, for example hydrolysis, hydrogenolysis, etc. after the reactions step is complete.

Salt and chelate formation may be effected by conventional techniques, e.g. as described in the above mentioned patent publications.

The skeletons of the macrocyclic chelant groups or, more preferably, of the linker moiety, may be derivatised to enhance properties of the overall chelant, for example to include hydrophilic or lipophilic groups or biologically targetting groups or structures. Examples of macromolecules, biomolecules and macrostructures to which the polymeric chelant may be conjugated in this way include polymers (such as polylysine or polyethyleneglycol), dendrimers (such as first to sixth generation starburst dendrimers and in particular PAMAM dendrimers), polysaccharides, proteins, antibodies or fragments thereof (especially monoclonal antibodies or fragments such as Fab fragments), glycoproteins, proteoglycans, liposomes, aerogels, peptides, hormones, steroids, microorganisms, human or non-human cells or cell fragments, cell adhesion molecules (in particular nerve adhesion molecules such as are described in WO-A-92/04916), other biomolecules, etc). Generally such derivatisation will be achieved most conveniently by the introduction of alkyl- or aralkyl-carried functions, to which the macromolecule, biomolecule, etc. can be bound either directly or via a linker molecule, for example a bi- or polyfunctional acid, activated acid or oxirane.

In the case of conjugation to dendrimers, the dendrimer carriers can be produced by standard techniques, for example as desribed by Tomalia and by Nycomed Salutar in Angew Chem Int Ed Eng 29:138 (1990), WO-A-88/01178, WO-A-90/12050 and WO-A-93/06868 and the references cited therein.

Such macromolecular derivatives of the compounds of formula V and the metal chelates and salts thereof form a further aspect of the present invention.

The linkage of a compound of formula V to a macromolecule or backbone polymer may be effected by the methods of Nycomed Salutar (WO-A-90/12050) or by any of the conventional methods such as the carbodiimide method, the mixed anhydride procedure of Krejcarek et al. (see Biochemical and Biophysical Research Communications 77: 581 (1977)), the cyclic anhydride method of Hnatowich et al. (see Science 220: 613 (1983) and elsewhere), the backbone conjugation techniques of Meares et al. (see Anal. Biochem. 142: 68 (1984) and elsewhere) and Schering (see EP-A-331616 for example) and by the use of linker molecules as described for example by Nycomed Imaging in WO-A-89/06979 (US-A-5208324).

Salt and chelate formation may be performed in a conventional manner. The chelating agents of formula V may be used in detoxification or in the formation of metal chelates, chelates which may be used for example in or as contrast agents for in vivo or in vitro magnetic resonance (MR), X-ray or ultrasound diagnostics (e.g. MR imaging and MR spectroscopy), or scintigraphy or in or as therapeutic agents for radiotherapy, and such uses of these metal chelates form a further aspect of the present invention.

Salts or chelate complexes of the compounds of the invention containing a heavy metal atom or ion are particularly useful in diagnostic imaging or therapy. Especially preferred are salts or complexes with metals of atomic numbers 20-32, 42-44, 49 and 57 to 83, especially Gd, Dy and Yb. For use as an MR-diagnostics contrast agent, the chelated metal species is particularly suitably a paramagnetic species, the metal conveniently being a transition metal or a lanthanide, preferably having an atomic number of 21-29, 42, 44 or 57-71. Metal chelates in which the metal species is Eu, Gd, Dy, Ho, Cr, Mn or Fe are especially preferred and Gd³⁺, Mn²⁺ and Dy³⁺ are particularly preferred.

Chelates of ions of these metals specifically listed above with chelants of formula V or their salts with physiologically tolerable counterions are particularly useful for the diagnostic imaging procedures mentioned herein and they and their use are deemed to fall within the scope of the invention and references to chelates of compounds of formula V herein are consequently to be taken to include such chelates.

The bislanthanide complexes of the compounds of formula V are especially preferred.

For diagnostic imaging purposes it is particularly important that the metal chelate complex be as stable as possible to prevent dissociation of the complex in the body.

In magnetic resonance imaging (MRI) it is frequently desirable to be able to target certain organs or tissues. In particular there is a need for improved hepatobiliary imaging MR contrast agents. Chelates of paramagnetic metals with compounds of formula V carrying one or more lipophilic groups are particularly suited for use as hepatobiliary MR contrast agents, since the presence of the lipophilic group will promote uptake by hepatocytes. By linking the lipophilic group to the molecule via a readily hydrolysable linking group such as an ester, the reabsorption after excretion to the intestine can be prevented.

The paramagnetic metal chelates of compounds of formula I, especially the chelates of high spin metal ions such as Gd³⁺ and more especially Dy³⁺, are particularly suitable for use as magnetic susceptibility (MS) contrast agents (T₂ or T₂* contrast agents) in MR imaging and other MR investigations. The use of paramagnetic metal mono-chelates is discussed by Villringer et al. in Mag. Reson. Med. 6:164-174 (1988) and by Kucharczyk et al. in US-A-5190744 and WO-A-91/14186. Using the dimeric chelates of the present invention, smaller volumes of contrast medium can be administered to achieve the same MS effect thus allowing for more effective bolus dosing. Moreover, while for monochelates high susceptibility paramagnetic centres such as Dy(III) generally have to be used in MS studies, by using the dimeric chelates of the invention a wider range of paramagnetic metal centres, including in particular Gd(III) become useful.

For certain hepatobiliary imaging purposes it is desirable that the lipophilic contrast agent be precipitated as particles which can be taken up by Kupffer cells in the liver. In such cases it is preferred to use chelates of Dy³⁺ with lipophilic compounds of formula V in conjunction with an imaging system utilising the magnetic susceptibility properties of the contrast agent; Kupffer cells in the liver are scarce and the contrast achievable using chelates with the gadolinium normally used in conventional MR imaging (i.e. as a T₁ relaxation agent) is generally insufficient. Such magnetic susceptibility agents form an important embodiment of the invention.

For use as contrast agents in MRI, the paramagnetic metal species is conveniently non-radioactive as radioactivity is a characteristic which is neither required nor desirable for MR-diagnostic contrast agents. For use as X-ray or ultrasound contrast agents, the chelated metal species is preferably a heavy metal species, for example a non-radioactive metal with an atomic number greater than 37, preferably greater than 50, e.g. Dy³⁺.

For use in scintigraphy and radiotherapy, the chelated metal species must of course be radioactive and any conventional complexable radioactive metal isotope, such as ^{99m}Tc, ⁶⁷Ga or ¹¹¹In for example, may be used. For radiotherapy, the chelating agent may be in the form of a metal chelate with for example ¹⁵³Sm, ⁶⁷Cu or ⁹⁰Y.

For use in detoxification of heavy metals, the chelating agent should be in salt form with a physiologically acceptable counterion, e.g. sodium, calcium, ammonium, zinc or meglumine, e.g. as the sodium salt of the chelate of the compound of formula V with zinc or calcium.

Where the metal chelate carries an overall charge, such as is the case with the prior art Gd DTPA, it will conveniently be used in the form of a salt with a physiologically acceptable counterion, for example an ammonium, substituted ammonium, alkali metal or alkaline earth metal (e.g. calcium) cation or an anion deriving from an inorganic or organic acid. In this regard, meglumine salts are particularly preferred.

The diagnostic and therapeutic agents of the present invention may be formulated with conventional pharmaceutical or veterinary formulation aids, for example stabilizers, antioxidants, osmolality adjusting agents, buffers, pH adjusting agents, etc. and may be in a form suitable for parenteral or enteral administration, for example injection or infusion or administration directly into a body cavity having an external escape duct, for example the gastrointestinal tract, the bladder or the uterus. Thus the agent of the present invention may be in a conventional pharmaceutical administration form such as a tablet, capsule, powder, solution, suspension, dispersion, syrup, suppository, etc; however, solutions, suspensions and dispersions in physiologically acceptable carrier media, for example water for injections, will generally be preferred.

The compounds according to the invention may therefore be formulated for administration using physiologically acceptable carriers or excipients in a manner fully within the skill of the art. For example, the compounds, optionally with the addition of pharmaceutically acceptable excipients, may be suspended or dissolved in an aqueous medium, with the resulting solution or suspension then being sterilized. Suitable additives include, for example, physiologically biocompatible buffers (as for example, tromethamine hydrochloride), additions (e.g. 0.01 to 10 mole percent) of chelants (such as, for example, DTPA, DTPA-bisamide or non-complexed chelants of formula I) or calcium chelate complexes (as for example calcium DTPA, CaNaDTPA-bisamide, calcium salts or chelates of chelants of formula VII), or, optionally, additions (e.g. 1 to 50 mole percent) of calcium or sodium salts (for example, calcium chloride, calcium ascorbate, calcium gluconate or calcium lactate combined with metal chelate complexes of chelants of formula I and the like).

If the compounds are to be formulated in suspension form, e.g., in water or physiological saline for oral administration, a small amount of soluble chelate may be mixed with one or more of the inactive ingredients traditionally present in oral solutions and/or surfactants and/or aromatics for flavouring.

For MRI and for X-ray imaging of some portions of the body the most preferred mode for administering metal chelates as contrast agents is parenteral, e.g. intravenous administration. Parenterally administrable forms, e.g. intravenous solutions, should be sterile and free from physiologically unacceptable agents, and should have low osmolality to minimize irritation or other adverse effects upon administration, and thus the contrast medium should preferably be isotonic or slightly hypertonic. Suitable vehicles include aqueous vehicles customarily used for administering parenteral solutions such as Sodium Chloride Injection, Ringer's Injection, Dextrose Injection, Dextrose and Sodium Chloride Injection, Lactated Ringer's Injection and other solutions such as are described in Remington's Pharmaceutical Sciences, 15th ed., Easton: Mack Publishing Co., pp. 1405-1412 and 1461-1487 (1975) and The National Formulary XIV, 14th ed. Washington: American Pharmaceutical Association (1975). The solutions can contain preservatives, antimicrobial agents, buffers and antioxidants conventionally used for parenteral solutions, excipients and other additives which are compatible with the chelates and which will not interfere with the manufacture, storage or use of products.

Where the diagnostic or therapeutic agent comprises a chelate or salt of a toxic metal species, e.g. a heavy metal ion, it may be desirable to include within the formulation a slight excess of the chelating agent, e.g. as discussed by Schering in DE-A-3640708 (US-A-5098692), or more preferably a slight excess of the calcium salt of such a chelating agent.

For MR-diagnostic examination, the diagnostic agent of the present invention, if in solution, suspension or dispersion form, will generally contain the metal chelate at concentration in the range 1 micromole to 1.5 mole per litre, preferably 0.1 to 700mM. The diagnostic agent may however be supplied in a more concentrated form for dilution prior to administration. The diagnostic agent of the invention may conveniently be administered in amounts of from 10⁻³ to 3 mmol of the metal species per kilogram of body weight, e.g. about 1 mmol lanthanide (e.g. Dy or Gd)/kg bodyweight.

For X-ray examination, the dose of the contrast agent should generally be higher and for scintigraphic examination the dose should generally be lower than for MR examination. For radiotherapy and detoxification, conventional dosages may be used.

The disclosures of all of the documents mentioned herein are incorporated by reference.

The present invention will now be illustrated further by the following non-limiting Examples. All ratios and percentages given herein are by weight and all temperatures are in degrees Celsius unless otherwise indicated.

### Example 1

### Synthesis of N,N'-Bis[1,4,7-tris-(carboxymethyl)-1,4,7,10-tetraazacyclo-dodecan-10-yl-methylcarbonyl]-piperazine and the gadolinium complex thereof

### (a) 1,4,7,10-Tetraazacyclododecane (cyclen)

To a suspension of tetraaza-12-crown-4 tetrahydrochloride (66.6 g, 0.209 mol, Parish, Inc.) in chloroform (2 L), was bubbled NH₃ (g) through a gas dispersion tube for 1 hour. The solution was allowed to stir overnight and the white solid was filtered off and washed with CHCl₃ (4 x 100 mL). The combined filtrate was concentrated in vacuo to a white solid which was washed with diethyl ether (4 x 50 mL) and dried under vacuum at ambient temperature. A second crop of the free base was isolated from the ether washes to give a combined yield of 35.1 g (97.5%). ¹H NMR (CDCl₃) : δ 2.32 (s, 4 H), 2.70 (s, 16 H).

### (b) 1,4,7-Tris-(tert-butoxy-carbonylmethyl)-1,4,7,10-tetraazacyclododecane-hydrobromide

Cyclen (35.0 g, 0.203 mole) (Example 1(a)) was dissolved in N,N-dimethylacetamide (DMA, 600 mL) under nitrogen. Sodium acetate (50.0 g, 0.61 mol) was added at once and the mixture was allowed to stir for 0.5 hour. A solution of t-butylbromoacetate (118.9 g, 0.61 mol) in DMA (150 mL) was added dropwise from an addition funnel over a 7 hour period. The reaction mixture was allowed to stir for 19 days at ambient temperature under nitrogen during which time a white solid precipitated from solution. The white solid was then filtered off, washed with chilled DMA (75 mL) and ethyl acetate (100 mL), and dried under vacuum at 50°C. The filtrate was concentrated to approximately 500 mL and a second crop of white solid was collected in a similar manner. The combined solids (80.2 g + 38.4 g) were taken up in CHCl₃ (600 mL) and washed with deionized H₂O (4 x 100 mL). The organic layer was dried (Na₂SO₄), filtered, and concentrated to a light yellow oil. A white solid was obtained by addition of ethyl acetate to the oil. The solid was collected by filtration, washed with diethyl ether (2 x 75 mL) , and dried under vacuum at 45°C to give 67.4 g (55.7%) of the title monohydrobromide salt. Additional product can be recovered from the filtrates if desired. ¹H NMR (CDCl₃): δ 1.42 (s, 27 H), 1.71 (s, 2 H), 2.86 (m, 12 H), 3.06 (br s, 4 H), 3.25 (s, 2 H), 3.34 (s, 4 H). Anal. Calcd (found) for C₂₆H₅₁N₄O₆Br: C, 52.43 (52.47); H, 8.63 (8.48); N, 9.40 (9.50); Br, 13.42 (12.92).

### (c) Piperazine bis(bromoacetamide)

To a 1 L three-necked flask equipped with a magnetic stir bar, reflux condenser and addition funnel, was added bromo acetyl bromide (82.8 g, 0.41 mole) in CHCl₃ (170 mL). The addition funnel was charged with a solution of piperazine (0.2 mole, 17.2 g) and triethylamine (70 mL) in CHCl₃ (180 mL). The flask was chilled to -15°C by means of a CH₃CN/liquid N₂ bath, and the amine was slowly added to the acid bromide. After the addition was complete the mixture was allowed to warm to ambient temperature and was stirred for 1 hour. The flask was then cooled to 0°C and H₂O (100 mL) was slowly added. The mixture was diluted with CHCl₃ (500 mL) and the layers were separated. The organic layer was extracted with H₂O (5 x 50 mL), 0.05 N NaOH (5 x 50 mL) and H₂O (200 mL), and dried (Na₂SO₄). The dark orange solution was filtered and concentrated to a beige solid. The material was purified by filtration through a bed of silica gel. The product eluted with 2 - 5% methanol/CH₂Cl₂. After combining and concentrating the desired fractions, 15.0 g (49.2%) of a white solid was obtained. The title product was recrystallized from warm 2-propanol (400 mL) affording 9.95 g. ¹H NMR (CDCl₃) : δ 3.61 (dt, 6 H), 3.85 (s, 4 H).

### (d) N,N'-Bis[1,4,7-tris-(tert-butoxycarbonylmethyl)-1,4,7,10-tetraazacyclododecan-10-yl-methylcarbonyl]-piperazine

To a solution of the hydrobromide salt of Example 1(b) (10.0 g, 16.8 mmol) in CHCl₃ (20 mL) and THF (80 mL) was added 1,1,3,3-tetramethylguanidine (TMG, 1.93 g 16.8 mmol). A white solid was produced which was filtered off, washed with 20% CHCl₃/THF (100 mL) and identified as TMG·HBr. The filtrate was concentrated to a clear oil which was dissolved in N,N-dimethylformamide (DMF, 200 mL) and treated with the bisbromoacetamide of Example 1(c) (2.62 g, 8.0 mmol) and TMG (1.93 g). The light yellow solution was warmed to 60°C and was allowed to stir for 16 hours under nitrogen. The reaction mixture was cooled to ambient temperature and the DMF was removed under vacuum. The residue was taken up in CH₂Cl₂ (300 mL) and was washed with 1 M Na₂CO₃ (3 x 60 mL). The combined aqueous layer was back-extracted with CH₂Cl₂ (50 mL). The combined CH₂Cl₂ layers were extracted with 1 M HCl (2 x 80 mL) followed by deionized H₂O (2 x 50 mL ). The combined HCl and H₂O layers were washed with CH₂Cl₂ (50 mL). The aqueous layer was combined with CH₂Cl₂ (250 mL) in an Erlenmeyer flask, and the pH was adjusted to 9 - 10 with anhydrous Na₂CO₃. The neutralized mixture was transferred to a separating funnel and the layers were separated. The aqueous layer was extracted with CH₂Cl₂ (2 x 100 mL), and all of the basic CH₂Cl₂ layers were combined and washed with H₂O (2 x 70 mL) . The organic layer was dried (Na₂SO₄), filtered, and concentrated to give 12.3 g of an off-white solid. The solid was triturated with ethyl acetate (50 mL) collected by filtration, washed with ethyl acetate (2 x 20 mL), diethyl ether (30 mL), and dried under vacuum to give 8.40 g (76 %) of a white solid. This material analyzed as the title dimer containing two NaX molecules (where X = Cl or Br). ¹H NMR (CD₃OD) : δ 1.39 (s, 54 H), 1.9 - 3.5 (br m, 56 H). ¹³C NMR (CD₃OD) : δ 174.5, 174.4, 172.2, 82.8, 82.6, 79.5, 56.7, 56.3, 54.0 (br), 49.0 (br), 45.2, 44.9, 42.9, 42.6, 28.5, 28.4. MS (FAB): m/e 1218 (MNa⁺). Anal: Calculated (found) for C₆₀H₁₁₀N₁₀O₁₄Na₂Cl₂·4H₂O: C, 52.04 (52.14); H, 8.59 (8.68); N, 10.12 (10.07); Na, 3.32 (3.44); Cl, 5.12 (5.16).

### (e) N,N'-Bis[1,4,7-tris-(carobyxmethyl)-1,4,7,10-tetraazacyclododecan-10-yl-methylcarbonyl]-piperazine

A solution of the dimer of Example 1(d) (10.0 g, 7.2 mol) in CH₂Cl₂ (120 mL) and trifluoroacetic acid (TFA, 80 mL) was allowed to stir at ambient temperature for 1 hour. The volatiles were removed by rotary evaporation to give a thick brown oil. The residue was redissolved in CH₂Cl₂ and trifluoroacetic acid as above for 1 hour. The process had to be repeated seven times to completely remove all of the t-butyl groups. After the final treatment with TFA, the crude product was concentrated, dissolved in H₂O (100 mL) and reconcentrated by rotary evaporation. The H₂O chase was repeated several times. The product was precipitated as a white solid by dissolving the crude mixture in H₂O (20 mL), warming the solution to 50°C and adding 2-propanol (150 mL) slowly. The solid was collected by filtration and washed with 2-propanol (2 x 50 mL) and acetone (2 x 30 mL). A second crop of product was collected from the filtrate and isolated in a similar fashion. The combined solids were dried under vacuum to give 7.48 g of material that contained traces of sodium and TFA. A portion of the solid, (5.24 g) was dissolved in H₂O (10 mL) and the pH was adjusted to 10.9 with 2 N NaOH. The solution was loaded onto a column containing Bio-Rad AG1-X8 (acetate form) and the column was washed with H₂O (2 L). The product was then eluted with 0.1 N acetic acid. After combining desired fractions and concentrating, the product was precipitated by dissolving in H₂O (50-60°C) and slowly adding 2:1 acetone/ethanol (60 mL). The solid was collected, washed with 2:1 acetone/ethanol (60 mL), and dried in vacuo to give 3.31 g (50 %) of the title product. ¹H NMR (NaOD, D₂O) : δ 2.20 (br s, 16 H), 2.46 (br s, 16 H), 2.88 (s, 12 H), 3.26 (d, 4 H), 3.40 (s, 8 H). ¹³C NMR (NaOD, D₂O) : δ 180.7, 180.6, 172.4, 59.3, 59.2, 54.9, 54.6, 51.2 (br), 44.6, 44.5, 41.9, 41.8. MS (FAB): m/e 859.5 (MH⁺). Anal: Calculated (found) for C₃₆H₆₂N₁₀O₁₄•4.5H₂O: C, 46.00 (46.00); H, 7.61 (7.45); N, 14.90 (15.01).

### (f) Bisgadolinium complex of N,N'-Bis[1,4,7-tris-(carboxymethyl)-1,4,7,10-tetraazacyclododecan-10-yl-methylcarbonyl]-piperazine

To a suspension of the dimeric chelant of Example 1(e) (5.65 g, 6.0 mmol) in deionized H₂O (120 mL) was added gadolinium acetate (4.73 g). A clear slightly yellow solution formed within minutes. The mixture was stirred at ambient temperature for 3 hours and was then concentrated by rotary evaporation (50°C) to drive off acetic acid. The residue was redissolved in H₂O (150 mL) and the solution was warmed to 40°C. No gadolinium was detected after 2 hours by a xylenol orange test. Gadolinium acetate was added in 12.2 mg (0.5 mol%) increments until a positive test for free gadolinium was observed. The solution was then treated with ligand to adjust the titer to ≤0.1 mol% excess ligand. The complex was precipitated by dissolving in H₂O (40°C) and adding a 2:1 acetone/ethanol solution. The solid was collected by filtration, washed with 2:1 acetone/ethanol (2 x 25 mL) and dried under vacuum at 35°C to give 6.4 g (80 %) of the title product. MS (FAB) : m/e 1168 (MH⁺). Anal: Calculated (found) for C₃₆H₅₆N₁₀O₁₄Gd₂•8.75H₂O: C, 32.64 (32.48); H, 5.58 (5.16); N, 10.57 (10.42); Gd, 23.73 (23.31).

### Example 2

### Synthesis of N,N'-bis[1,4,7-tris-(carboxymethyl)-1,4,7,10-tetraazacyclododecan-10-yl-methylcarbonyl]-N,N'-dimethylethylenediamine and the gadolinium complex thereof

### (a) N,N'-Dimethylethylenediamine bis (bromoacetamide)

To a 1 L three-necked flask equipped with a magnetic stir bar, reflux condenser and addition funnel, was added bromoacetyl bromide (46.95 g , 232.6 mmole) and CHCl₃ (100 mL). The addition funnel was charged with a solution of N,N'-dimethylethylenediamine (10.0 g, 113.4 mmol) and triethylamine (39.5 mL) in CHCl₃ (100 mL). The flask was chilled to -15°C by means of an ethylene glycol/CO₂ bath, and the amine was slowly added to the acid bromide. After the addition was complete, the mixture was allowed to warm to ambient temperature and stirred for 1 hour. The flask was then cooled to 0°C and H₂O (50 mL) was slowly added. The mixture was diluted with CHCl₃ (250 mL) and the layers were separated. The organic layer was extracted with H₂O (3 x 50 mL), 0.05 N NaOH (3 x 50 mL), and H₂O (2 x 50 mL), and was dried (Na₂SO₄). The solution was filtered and concentrated. The material was methanol purified by filtration through a bed of silica gel. The product eluted with 2 - 5% methanol/CH₂Cl₂. After combining and concentrating the desired fractions, 14.95g (39.9%) of a beige solid was obtained. The title product was recrystallized from warm 2-propanol affording 11.94 g. ¹H NMR (CDCl₃) : δ [2.99 (s), 3.11 (s), 3.13 (s); 6 H], [3.53 (s), 3.57 (s); 4 H], 3.81 (s), 3.84 (s), 3.91 (s); 4 H].

### (b) N,N'-Bis[1,4,7-tris-(tert-butoxycarbonylmethyl)1,4,7,10-tetraazacyclododecan-10-yl-methylcarbonyl]-N,N'-dimethylethylenediamine

To a solution of the hydrobromide salt of Example 1(b) (8.0 g, 13.4 nmol) in CHCl₃ (20 mL) and THF (80 mL) was added 1,1,3,3-tetramethylguanidine (TMG, 1.547 g, 13.4 mmol). A white solid was produced which was filtered off, washed with 20% CHCl₃/THF (100 mL) and identified as TMG•HBr. The filtrate was concentrated to a clear oil which was dissolved in N,N-dimethylformamide (DMF, 200 mL) and treated with the bis(bromoacetamide) of Example 2(a) (2.21 g, 6.7 mmol) and TMG (1.547 g). The light yellow solution was warmed to 60°C and was allowed to stir for 16 hours under nitrogen. The reaction mixture was cooled to ambient temperature and the DMF was removed under vacuum. The residue was taken up in CHCl₃ (200 mL) and washed with 1 M Na₂CO₃ (3 x 40 mL). The combined aqueous layer was back-extracted with CHCl₃ (50 mL). The combined CHCl₃ layers were extracted with 0.8 M HCl (2 x 50 mL) followed by deionized H₂O (2 x 50 mL). The combined HCl and H₂O layers were washed with CHCl₃ (50 mL). The aqueous layer was combined with CHCl₃ (200 mL) in an Erlenmeyer flask, and the pH was adjusted to between 9.5 with Na₂CO₃. The neutralized mixture was transferred to a separating funnel and the layers were separated. The aqueous layer was extracted with CHCl₃ (2 x 100 mL) , and the basic CHCl₃ layers were combined and washed with H₂O (2 x 50 mL). The organic layer was dried over anhydrous Na₂SO₄, filtered and concentrated to give 7.50 g (93 %) of the title product as a yellow oil. ¹H NMR (CDCl₃): δ 1.24 (s, 54 H), 1.90-3.39 (br m, 67 H). ¹³C NMR (CDCl₃): δ 27.7, 27.8, 34.9. 45.7, 48.3 (br), 52.0 (br), 55.4, 55.6, 56.3, 81.4, 81.6, 81.6, 81.7, 81.8, 171.5, 172.6, 172.7.

### (c) N,N'-Bis[1,4,7,10-tris-(carboxymethyl)-1,4,7,10-tetraazacyclododecan-10-yl-methylcarbonyl]-N,N'-dimethylethylenediamine

A solution of the dimer of Example 2(b) (7.50 g) in CH₂Cl₂ (100 mL) and trifluoroacetic acid (TFA, 60 mL) was allowed to stir at ambient temperature for 1 hour. The volatiles were removed by rotary evaporation to give a thick brown oil. The residue was redissolved in CH₂Cl₂ (20 mL) and trifluoroacetic acid (15 mL) as above for 1 hour. The process was repeated seven times to completely remove all of the t-butyl groups. After the final treatment with TFA, the crude product was concentrated, dissolved in H₂O (100 mL) and reconcentrated by rotary evaporation. The H₂O chase was repeated several times. The product was purified by ion-exchange chromatography (Bio Rad AG1-X8, acetate form) using 0.1 N acetic acid to elute the product. Desired fractions were combined and concentrated to give 2.36 g (37 %) of the title product as an off white solid after lyophilization. ¹H NMR (NaOD, D₂O) : δ 2.0-3.7 (br, m). ¹³C NMR (NaOD, D₂O) : δ 34.5, 35.4 (br), 43.7, 45.6, 47.2, 47.2, 48.9, 49.6, 50.0, 51.6 (br), 52.5, 55.1, 56.0, 57.3, 59.1, 63.3, 173.4 (br), 179.2, 180.5, 181.4. MS (FAB): m/e 861 (MH⁺), 883 (MNa⁺), 917 (M + NaCl - H)⁺], 973 [(M + 2NaCl -2H)⁺].

### (d) Bisgadolinium complex of N,N'-Bis[1,4,7-tris-(carboxymethyl)-1,4,7,10-tetraazacyclododecan-10-yl-methylcarbonyl]-N,N'-dimethyl-ethylenediamine

To a solution of the dimeric chelant of Example 2(c) (1.88 g, 2.0 mmol) in deionized H₂O (40 mL) was added gadolinium acetate (1.53 g). The light yellow solution was stirred at 40°C for 1 hour and was then concentrated by rotary evaporation (50°C) to drive off acetic acid.

The residue was redissolved in H₂O (100 mL) and the solution was warmed to 40°C. The reaction was allowed to stir overnight at ambient temperature. Gadolinium acetate was added in 0.01 mmol increments until a positive test for free gadolinium was observed. The solution was then treated with ligand to adjust the titer to 0.1 mol% excess ligand. The solution was concentrated to dryness and was triturated with 2:1 diethylether/CHCl₃ to give the title product as a beige solid (2.57 g, 98 %).

### Example 3

### Synthesis of N,N'-Bis[1,4,7-tris- (carboxymethyl)-1,4,7,10-tetraazacyclododecan-10-yl-methylcarbonyl]tetrahydroquinoxaline and the gadolinium complex thereof

### (a) Tetrahydroquinoxaline bis(chloroacetamide)

To a 1 L three-necked round bottomed flask equipped with a magnetic stir bar, reflux condenser and addition funnel, was added chloroacetyl chloride (9.6 mL, 0.120 mol) and CHCl₃ (100 mL). The addition funnel was charged with a solution of tetrahydroquinoxaline (8.0 g, 0.0596 mol) and triethylamine (20.8 mL) in CHCl₃ (100 mL). The flask was chilled to 0°C, and the amine was slowly added to the acid chloride. After the addition was complete, the mixture was allowed to warm to ambient temperature and stir for 1 hour. The flask was then cooled to 0°C and H₂O (50 mL) was slowly added. The mixture was diluted with CHCl₃ (250 mL) and the layers were separated. The organic layer was washed with H₂O (50 mL), 0.05 N NaOH (2 x 50 mL), H₂O (50 mL), 1 N HCl (3 x 50 mL) and H₂O (2 x 50 mL), and was dried over anhydrous Na₂SO₄. The solution was filtered and concentrated to a brown solid. The solid was collected by filtration, washed with 2-propanol (2 x 30 mL) and diethylether (2 x 40 mL) and dried to yield the title product (4.41 g, 51.5%). ¹H NMR (DMSO-d₆): δ 3.89 (s, 4 H), 4.01 (s, 4 H), 7.25 (s, 2 H), 7.65 (br s, 2 H).

### (b) N,N'-Bis[1,4,7-tris-(tert-butoxycarbonylmethyl)-1,4,7,10-tetraazacyclododecan-10-yl-methylcarbonyl]tetrahydroquinoxaline

To a solution of the hydrobromide salt of Example 1(b) (8.0 g, 13.4 mmol) in CHCl₃ (20 mL) and THF (80 mL) was added 1,1,3,3-tetramethylguanidine (TMG, 1.547 g, 13.4 mmol). A white solid was produced which was filtered off, washed with 20% CHCl₃/THF (100 mL) and identified as TMG•HBr. The filtrate was concentrated to a clear oil which was dissolved in N,N-dimethylformamide (DMF, 250 mL) and treated with bis(chloroacetamide) of Example 3(a) (1.928 g, 6.72 mmol) and TMG (1.547 g). The light yellow solution was warmed to 60°C and was allowed to stir for approximately 16 hours under nitrogen. The reaction mixture was cooled to ambient temperature and the DMF was removed under vacuum. The residue was taken up in CHCl₃ (200 mL) and was washed with 1 M Na₂CO₃ (3 x 40 mL). The combined aqueous layer was back-extracted with CHCl₃ (50 mL). The combined CHCl₃ layers were extracted with 1 M HCl (2 x 50 mL) followed by deionized H₂O (2 x 50 mL). The combined HCl and H₂O layers were washed with CHCl₃ (2 x 50 mL). The aqueous layer was combined with CHCl₃ (250 mL) in an Erlenmeyer flask, and the pH was adjusted to 9.5 (Na₂CO₄). The neutralized mixture was transferred to a separating funnel and the layers were separated. The aqueous layer was extracted with CHCl₃ (2 x 50 mL), and all CHCl₃ layers were combined and washed with brine (2 x 40 mL). The organic layer was dried (Na₂SO₄), filtered, and concentrated to give 10.60 g of a yellow oil. The title product was purified further by passing through a bed of silica gel and eluting the product with 10% methanol/CHCl₃ to give 7.06 g (84.6 %). ¹H NMR (CDCl₃): δ 1.30 (s), 1.38 (s), 1.41 (s), 2.0-3.7 (br m), 7.28 (s) .

### (c) N,N'-Bis[1,4,7-tris-(carboxymethyl)-1,4,7,10-tetraazacyclododecan-10-yl-methylcarbonyl]tetrahyquinoxaline

A solution of the dimer of Example 3(b) (7.06 g) in CH₂Cl₂ (70 mL) and trifluoroacetic acid (TFA, 60 mL) was allowed to stir at ambient temperature for 1 hour. The volatiles were removed by rotary evaporation to give a thick brown oil. The residue was redissolved in CH₂Cl₂ (70 mL) and trifluoroacetic acid (60 mL) as above for 1 hour. The process was repeated nine times to completely remove all of the t-butyl groups. After the final treatment with TFA, the crude product was concentrated, dissolved in H₂O (100 mL), and reconcentrated by rotary evaporation. The H₂O chase was repeated several times. The product was purified by ion-exchange chromatography column (Bio Rad AG1-X8, acetate form) using 0.1 N acetic acid to elute the product. Desired fractions were combined and concentrated to give 3.26 g of an off white solid after lyophilization The solid was triturated with ethanol (50 mL), collected by filtration, washed with diethyl ether and dried to yield the title product (3.18 g, 48 %). ¹H NMR (NaOD, D₂O) : δ [2.88 (br s), 3.19 (br s), 3.43 (s), 3.53-3.68 (m); 56 H], 7.07 (m, 3 H), 7.59 (br s, 1 H).

### (d) Bisgadolinium complex of N,N'-bis[1,4,7-tris (carboxymethyl)-1,4,7,10-tetraazacyclododecan-10-yl-methylcarbonyl]-tetrahyquinoxaline

To a solution of the dimeric chelant of Example 3(c) (2.50 g, 2.53 mmol) in 100 mL deionized H₂O (100 mL) was added gadolinium acetate (1.9827 g, 96% of theroretical). The clear solution was stirred at 40°C for 1 hour and was then concentrated by rotary evaporation (50°C) to drive off acetic acid. The residue was redissolved in H₂O (100 mL) and the solution was warmed to 40°C and the reaction allowed to stir overnight at ambient temperature. Gadolinium acetate was added in 0.5 mol% increments until a positive test for gadolinium was observed. The solution was then treated with ligand to adjust the titer to 0.5 mol% excess ligand. The solution was lyophlized to give 3.28 g of the title product as an off-white solid. Mass spectrum (FAB): m/e 1217 (MH⁺).

### Example 4

### Synthesis of N,N'-Bis[1,4,7-tris-(carboxymethyl)-1,4,7,10-tetraazacyclododecan-10-yl-methylcarbonyl]ethylenediamine and the gadolinium complex thereof

### (a) Ethylenediamine bis(chloroacetamide)

To a solution of ethylenediamine (3.0 g, 0.05 mol) in CHCl₃ (50 mL) cooled to -15°C was added chloroacetyl chloride (7.96 mL, 0.10 mol) in CHCl₃ (40 mL) dropwise. A white precipitate formed and the reaction mixture was stirred overnight at ambient temperature. After 16 hours the reaction mixture was filtered and the white solid was washed with CHCl₃ (30 mL) followed by water (100 mL). The CHCl₃ layer was washed with water (2 x 25 mL), dried (Na₂SO₄), and filtered. After being dried under vacuum, the white solid was recrystallized from ethanol and filtered hot to give a clear filtrate from which the product crystallized upon standing. The solid was collected by filtration and washed with isopropyl alcohol (20 mL). The mother liquor from the recrystallization and the CHCl₃ layer were combined and concentrated yielding a white solid which was recrystallized from ethanol and then washed with isopropyl alcohol. The title product (4.9 g, 46%) was obtained as a white solid. ¹H NMR (CDCl₃) δ 7.05 (br, 2 H), 4.04 (s, 4 H), 3.49 (s, 4 H). ¹³C NMR (DMSO-d₆) δ 166.2, 42.7, 38.5.

### (b) N,N'-Bis[1,4,7-tris-(tert-butoxycarbonylmethyl)-1,4,7,10-tetraazacyclododecan-10-yl-methylcarbonyl]-ethylenediamine

The hydrobromide salt of Example 1(b) (4.7 g, 7.9 mmol) was dissolved in 20% CHCl₃/THF (50 mL). To this solution was added TMG (0.99 mL, 7.9 mmol) with the solution quickly becoming cloudy as TMG·HBr precipitated. After stirring for 15 minutes, the solution was filtered and the filtrate concentrated to a yellow oil. The oil was taken up in THF (80 mL) and transfered to a three necked round bottom flask. The flask was then charged with NaI (590 mg, 3.9 mmol), TMG (0.99 mL, 7.9 mmol), and finally the bischloroacetamide of Example 4(a) (837 mg, 3.93 mmol). The reaction mixture was placed under N₂ and heated to 68°C. After 16 hours, the reaction mixture was filtered and the filtrate concentrated to a gummy solid. The residue was taken up in CH₂Cl₂ and washed with 1.0 N Na₂CO₃ (3 x 50 mL). The aqueous phase was back extracted with CH₂Cl₂. The organic portions were combined and extracted with 1 M HCl (1 x 80 mL). The aqueous phase was then washed with CH₂Cl₂ and the pH of the aqueous phase was adjusted to 9 with Na₂CO₃. The aqueous layer was then extracted with CH₂Cl₂ (1 x 200 mL, 2 x 100 mL) and the CH₂Cl₂ layer washed with water (2 x 75 mL), dried (MgSO₄), and filtered. Concentration and drying under vacuum yielded 3.6 grams (79%) of the title compound as a pale yellow solid. ¹H NMR (CDCl₃) δ 3.34 - 2.00 (br band, 57 H), 1.44 (br s, 54 H).

### (c) N,N'-Bis[1,4,7-tris-(carboxymethyl)-1,4,7,10-tetraazacyclododecan-10-yl-methylcarbonyl]-ethylenediamine

The ethylenediamine dimer of Example 4(b) (6.7 g; 5.7 mmol) was dissolved in CH₂Cl₂ (40 mL) and a 1:1 solution of TFA/CH₂Cl₂ (20 mL) was added. The resulting mixture was stirred for one hour and then concentrated. This procedure was repeated eight times to complete the deprotection. After the final deprotection, the solution was concentrated and chased with water (3 x 25 mL) to yield a 6.2 grams of a brownish solid. The solid was dissolved in water (15 mL), the pH was adjusted to 10.9 with 3 N LiOH, and the solution was loaded onto AG1-X8 ion exchange resin (acetate form). The column bed was washed with water (1 L), and the dimer eluted from the column with 0.1 N acetic acid with the desired fractions being combined and concentrated yielding 2.5 g (51%) of the title product as a pale yellow solid. ¹H NMR (D₂O) δ 3.62 - 2.89 (br band).

### (d) Bisgadolinium complex of N,N'-bis[1,4,7-tris-(carboxymethyl)-1,4,7,10-tetraazacyclododecan-10-yl-methylcarbonyl]ethylenediamine

The dimeric chelant of Example 4(c) (2.12 g, 2.32 mmol) was dissolved in water (50 mL) and gadolinium acetate (1.81 g, 2.25 mmol) was added. The mixture was allowed to stir at ambient temperature for 1.5 hours, the solution was concentrated, and the residue was taken up in water (50 mL). After another 1.5 hours, the solution gave a positive xylenol orange test, therefore the solution was concentrated and chased with water (1x 20 mL). The residue was taken up in water (50 mL) and allowed to stir overnight at ambient temperature. After stirring for 16 hours, the solution was chased with water to remove the acetic acid formed. Additional ligand was added in 21 mg (0.07 mmol) portions until the xylenol orange test gave a positive (purple) color. A final portion of 21 mg of ligand was added and the solution was stirred for two hours at 40°C. A xylenol orange test was performed and gave a negative result. The solution was then concentrated to give a pale yellow solid. The solid was dissolved in warm water (15 mL) to which was added a 2:1 acetone/ethanol solution (85 mL) from which a white solid precipitated. The solid was collected by filtration and washed with 2:1 acetone/ethanol solution (200 mL) followed by acetone (30 mL) and dried in a vacuum oven (35°C ) for 3.5 hours to yield 2.4 g of the title product as a white solid. MS (FAB): m/e 1143.2 (MH⁺). Anal: Calculated for C₃₄H₅₄Gd₂N₁₀O₁₄^{•}3.4H₂O: C,29.53; H,5.89; Gd,22.74; N,10.13. Found C,29.47; H,5.59; Gd,22.54; N,9.81.

### Example 5

### Synthesis of N,N'-bis[1,4,7-tris-(carboxymethyl)-1,4,7,10-tetraazacyclododecan-10-yl-methylcarbonyl]homopiperazine and the gadolinium complex thereof

### (a) Homopiperazine bis (chloroacetamide)

Homopiperazine (10 g; 100 mmol) was dissolved in chloroform (150 mL) and triethylamine (28 mL; 200 mmol) was added to the mixture. The solution was cooled to - 30'C under nitrogen and chloroacetyl chloride (17 mL, 213 mmol) was added over a period of 1 hour. The solution was stirred at -30°C for 1hour, at 0°C for 2 hour and at ambient temperature overnight. The solution was washed with water (4 x 50 mL), dried over anhydrous MgSO₄, filtered and concentrated. The oily residue was purified by column chromatography on silica gel (5 % methanol in chloroform) to yield the title product as an oil which solidified on prolonged standing (10.3 g; 41 %). ¹H NMR (CDCl₃) : 4.04 (m, 4 H), 3.62 (m, 8 H), and 1.94 (m, 2 H).

### (b) N,N'-Bis[1,4,7-tris-(tert-butoxycarbonylmethyl)-1,4,7,10-tetraazacyclododecan-10-yl-methylcarbonyl]homopiperazine

A solution of the bis(chloroacetamide) of Example 5(a) (1.7 g, 6.72 mmol), D03A-tri-t-butyl ester (8 g, 13.43 mmol) and tetramethylguanidine (2.6 mL, 20.75 mmol) in acetonitrile (300 mL) was heated at 60°C for 25 hours under nitrogen. The solvent was removed under reduced pressure and the residue redissolved in chloroform (60 mL). The solution was washed with 1 M sodium carbonate (2 x 50 mL) arid water (2 x 50 mL) and extracted with 1 M HCl (4 x 50 mL) followed by water (2 x 50 mL). The aqueous extracts were combined and basified with solid sodium carbonate. The crude product that separated as an oil was extracted with chloroform (3 x 100 mL), dried over anhydrous magnesium sulfate, filtered, and concentrated to yield the crude title product as a yellow solid (9.77 g) . ¹H NMR (CDCl₃) : 3.44-2.45 (m, 56 H), 1.84 (s, 2 H), 1.42 (s, 18 H), 1.40 (s, 36 H).

### (c) N,N'-Bis[1,4,7-tris-(carboxymethyl)-1,4,7,10-tetraazacyclododecan-10-yl-methylcarbonyl]homopiperazine

A solution of the crude t-butyl ester of Example 5(b) (9.77 g) in CH₂Cl₂ (30 mL) was treated with trifluoroacetic acid (30 mL) and the mixture stirred at ambient temperature for 1 hour. The solution was concentrated and the process repeated seven times. The oily residue (10.5 g) obtained after the final deprotection was dissolved in acetone (30 mL) and was precipitated with chloroform. Filtration and drying under vacuum yielded a yellow solid (8.33 g). The solid was dissolved in water (20 mL) and the pH of the solution was adjusted to 11.0 with 3.0 N LiOH. The solution was loaded onto an AG1-X8 ion exchange column (acetate form) and the column bed was washed with water (1 L). The desired dimer was eluted with 0.1 N acetic acid with the desired fractions being combined and and concentrated. Lyophilzation of the product yielded 3.8 g (54 %) of the title product as a yellow solid. ¹H NMR (D₂O) δ 3.63 - 2.74 (br band, 56 H), 1.6 ( br s, 2 H)

### (d) Bisgadolinium complex of N,N'-bis[1,4,7-tris-(carboxymethyl)-1,4,7,10-tetraazacyclododecan-10-yl-methylcarbonyl]homopiperazine

To the dimeric chelant of Example 5(c) (3.11g, 3.26 mmol) dissolved in water (60 mL) was added gadolinium acetate (2.52 g, 6.30 mmol). The solution was warmed to 40°C for one hour and then concentrated to remove the acetic acid formed. Water (60 mL) was again added, the solution was stirred for one hour, and the process to remove the acetic acid repeated. The solid was then taken up in water (60 mL) and stirred overnight at ambient temperature. The solution gave a weakly positive xylenol orange test, therefore ligand (31 mg, 0.07 mmol) was added. After 1.5 hours at 40°C the reaction mixture gave a negative xylenol orange test. The solution was concentrated and then dissolved in ethanol. Acetone was added and the precipitated complex collected by filtration. The solid was taken up in acetone and stirred for 1 hour and then collected by filtration. The solid was dried in a vacuum oven (35°C) for two hours to give 3.2 g (76 %) of the title product as a fine white powder. MS (FAB): m/e 1183.3 (MH⁺). Anal: Calculated for C₃₇H₅₈Gd₂N₁₀O₁₄^{•}12.3 H₂O^{•}0.72 acetone: C,32.55; H,6.06; Gd,21.77; N,9.69. Found C,32.92; H.5.69; Gd,21.47; N,9.91.

### Example 6

### Synthesis of N,N'-bis[1,4,7-tris-(carboxymethyl)-1,4,7,10-tetraazacyclododecan-10-yl-methylcarbonyl]-2,2'-(ethylenedioxy)diethylamine and the gadolinium complex thereof

### (a) 2,2'-(Ethylenedioxy)diethylamine bis(chloroacetamide)

To a solution of 2,2'(ethylenedioxy)diethylamine (10.0 g, 0.0675 mol) in CH₂Cl₂ (190 mL) was added Na₂CO₃ (14.2 g, 0.135 mol). The resulting mixture was chilled in an ice bath and placed under a stream of N₂ to which was added chloroacetyl chloride (10.7 mL, 0.135 mol) in CH₂Cl₂ dropwise over a period of 25 minutes. Upon completion of addition, the ice bath was removed and the mixture was stirred at ambient temperature for two hours. The reaction mixture was then filtered and the filtrate was washed with water (150 mL), saturated NaHCO₃ (150 mL), and finally water (150 mL). The organic phase was dried (over Mg₂SO₄), filtered, and concentrated to yield a yellow oil which solidified when placed under vacuum. The solid was triturated with a 1:1 mixture of ethyl acetate/hexane (150 mL). A white solid was collected by filtration and dried in a vacuum oven (50°C) for 6 hours yielding 6.9 g (34%) of the title product. ¹H NMR (CDCl₃) δ 6.97 (br s, 2 H), 4.03 (s, 4 H), 3.60 (s, 4 H), 3.56 (distorted t, 4 H), 3.50 (t, 4 H) ; ¹³C NMR (CDCl) δ 165.9, 70.1, 69.1 42.4, 39.3.

### (b) N,N'-Bis[1,4,7-tris-(tert-butoxycarbonylmethyl)-1,4,7,10-tetraazacyclododecan-10-yl-methylcarbonyl]-2,2'-(ethylenedioxy)diethylamine

The hydrobromide salt of Example 1(b) (19.8 g, 33.2 mmol) was dissolved in 20% CHCl₃/THF (250 mL). To this solution was added TMG (4.16 mL, 33.2 mmol) and the solution quickly became cloudy as TMG^{•}HBr precipitated. After stirring for 20 minutes, the solution was filtered with the filtrate being concentrated to a thick yellow oil. The residue was taken up in THF (100 mL) and transferred to a 1 L flask which was then charged with NaI (2.48 g, 16.6 mmol), TMG (4.16 ml, 33.2 mmol), and finally the bis(chloroacetamide) of Example 6(a) (5.0 g, 16.6 mmol). Additional THF (150 mL) was added, the system was placed under a stream of N₂, and the reaction mixture was heated to 65°C. After four days, the reaction mixture was filtered and the filtrate concentrated to a reddish solid. The solid was taken up in CH₂Cl₂ (110 mL) and washed with water (3 x 100 mL). The organic portion was dried (MgSO₄), filtered and concentrated to yield 22.14 g (114%) of the title product as a tacky yellow solid which was not analyzed for salts. ¹H NMR (CDCl₃) δ 3.39-2.06 (br band, 62 H), 1.25 (br s, 54 H) .

### (c) N,N'-Bis[1,4,7-tris-(carboxymethyl)-1,4,7,10-tetraazacyclododecan-10-yl-methylcarbonyl]-2,2'-(ethylenedioxy)diethylamine

The crude dimer of Example 6(b) (22.0 g, 18.7 mmol) was dissolved in CH₂Cl₂ (25 mL). To this solution was added a mixture of TFA (25 mL) and CH₂Cl₂ (10 mL). After stirring for 2 hours at ambient temperature, the reaction was concentrated. This procedure was repeated seven times to complete the deprotection. After the final deprotection, the solution was concentrated and chased with water (3 x 40 mL). The reddish semi-solid was then dissolved in water (30 mL) and the pH adjusted to 2.3 with 3.0 N NH₄OH. The solution was loaded onto an AG50-X8 ion-exchange resin (H⁺ form) and the column bed washed with water (1.8 L). The dimer was eluted from the column with 0.5 N NH₄OH with the desired fractions being combined and concentrated to yield 9.8 g (57 %) of the title product as a yellow solid. ¹H NMR (D₂O) δ 3.61-2.92 (br band) . MS (FAB) : m/e 921.4 (MH⁺).

### (d) Bisgadolinium complex of N,N'-bis[1,4,7-tris-(carboxymethyl)-1,4,7,10-tetraazacyclododecan-10-yl-methylcarbonyl]-2,2'-(ethylenedioxy)diethylamine

To a solution of the DO3A dimer of Example 6(c) (5.90 g, 5.94 mmol) in water (50 mL) was added gadolinium acetate (3.61 g, 8.91 mmol) and the reaction mixture was warmed to 40°C for 1.5 hours at which time the xylenol orange test was negative. Gadolinium acetate (240 mg, 0.594 mmol) was added in increments until a positive (purple) xylenol orange was achieved. Ligand was then added to achieve a negative xylenol orange test. The solution was reduced in volume and lyophilized yielding 7.58 g of crude complex. The crude complex was purified by HPLC employing a reverse phase C18 column using 2% methanol/water as the mobile phase. MS (FAB): m/e 1231.0 (MH⁺) Anal: Calculated for C₃₈H₆₂Gd₂N₁₀O₁₆^{•}7.65H₂O: C,33.38; H,5.70; Gd,23.00; N,10.24. Found C,33.48; H,5.79; Gd,22.75; N,10.50.

### Example 7

### Synthesis of N,N'-bis[1,4,7-tris-(carboxymethyl)-1,4,7,10-tetraazacyclododecan-10-yl-methylcarbonyl]-N,N'-bis(2,3-dihydroxypropyl)-ethylenediamine and the gadolinium complex thereof

### (a) 1,2,5,6-Di-isopropylidene-D-mannitol

To a solution of (411 g, 3.01 mol) of ZnCl₂ in 1.5 L of acetone cooled to 0°C was added (250 g, 1.37 mol) of D-mannitol and the mixture stirred at ambient temperature for 5 hours. The solid was filtered off and the filtrate poured into a solution of diethyl ether (1.8 L) and potassium carbonate (470 g, 3.4 mol) producing vigorous bubbling. The solution was stirred for 1 hour. A white precipitate was filtered off and the filtrate was dried (K₂CO₃), and concentrated leaving a white solid which was recrystallized from n-butyl ether to give 160 g (50%) of the title product as white needles. ¹H NMR (CDCl₃ ) δ 4.2 (m, 4 H), 4.0 (t, 2 H), 3.7 (d, 2 H), 2.7 (s, 2 H), 1.4 (d, 12 H).

### (b) Isopropylidene-glyceraldehyde

The mannitol derivative of Example 7(a) (81 g, 0.311 mol) was suspended in CH₂Cl₂ (1.1 L) and saturated NaHCO₃ (40 mL) and stirred at ambient temperature. Sodium periodate (100 g, 0.47 mol) was added in four portions over 20 minutes and the solution was stirred vigorously at 0°C for 3 hours. The solvent was decanted off and the remaining solid was stirred in CH₂Cl₂ and then filtered to remove the excess solid. The two organic portions were combined and concentrated *in vacuo*. The resulting oil was distilled at reduced pressure to give the title product as a clear, viscous oil. Yield (59 g, 75%). ¹H NMR (CDCl₃) δ 4.3 (t, 1 H), 3.9 (m, 2 H), 1.4 (d, 6 H).

### (c) N,N'-Bis(2,3-dihydroxypropyl)ethylenediamine bisacetonide

Ethylenediamine (13.2 g, 0.22 mol) was dissolved in methanol (150 mL) and the solution was adjusted with a 1:1 HCl/methanol mixture to pH 7. The solution was cooled with an ice bath and isopropylidene-glyceraldehyde of Example 7(b) (59 g, 0.45 mol) was added followed by portionwise addition of NaCNBH₃ (28.3g, 0.45 mol). The reaction mixture was stirred at 25°C under N₂ for 72 hours. The pH of the solution was then lowered to 3 with HCl/methanol and the solvent was stripped off in vacuo. The solid was taken up in H₂O and extracted with diethyl ether (3 x 200 mL). The pH of the aqueous phase was raised to 11 and extracted again with diethyl ether (4 x 200 mL). The ether washes were combined, dried (MgSO₄) and concentrated to a yellow oil. The title product was isolated by flash chromatography. (5% methanol/CHCl₃). Yield (37 g, 58%). ¹H NMR (CDCl₃) δ 4.1 (m, 4 H), 3.3 (m, 2 H), 2.6 (m, 8 H), 1.4 (d,12 H).

### (d) N,N'-Bis(2,3-dihydroxypropyl)ethylenediamine bisacetonide bischloroacetamide

The compound of Example 7(c) (37 g, 0.13 mol) and triethylamine (25.96 g, 0.25 mol) were combined in CH₂Cl₂ (300 mL). Chloroacetyl chloride (28.9 g, 0.25 mol) was added dropwise at 0°C under N₂. A color change was observed along with a white precipitate as the reaction mixture was allowed to return to ambient temperature. After 24 hours, H₂O (150 mL) was added, the organic layer was separated, washed with water (3 x 100 mL), dried (MgSO₄) and concentrated to a dark viscous oil. The title product (23 g, 41%) was isolated by flash chromatography, 10% methanol/CHCl₃. MS (FAB): m/e 442 (MH⁺)

### (e) N,N'-Bis[1,4,7-tris-(tert-butoxycarbonylmethyl)-1,4,7,10-tetraazacyclododecan-10-yl-methylcarbonyl]N,N'-bis(2,3-dihydroxypropyl)-ethylenediamine bisacetonide

The hydrobromide salt of Example 1(b) (12 g, 0.02 mol) and the compound of Example 7(d) (4.5 g, 0.01 mol) were dissolved in CH₃CN (300 mL) and tetramethylguanidine (7.6 mL, 0.61 mol). The reaction mixture was heated to 60°C and stirred under N₂ for 6 days. The solvent was stripped off and the resulting dark oil taken up in CHCl₃ and extracted with water (3 x 100 mL), dried (MgSO₄) and concentrated to give (12 g, 80%) of the title product MS(FAB): m/e 1398 (MH⁺).

### (f) N,N'-Bis[1,4,7-tris-(carboxymethyl)-1,4,7,10-tetraazyclododecan-10-yl-methylcarbonyl]N,N'-bis(2,3-dihydroxypropyl)ethylenediamine

The dimer of Example 7(e) was dissolved in CHCl₃ (175 mL) and trifluoroacetic acid (175 mL) was added dropwise. The reaction mixture was allowed to stir for 1 hour at ambient temperature under N₂ and then concentrated in vacuo to a dark oil. The acid treatment was repeated ten times to remove all t-butyl groups and the acetonides. The solvent was stripped off and the title product purified via preparative HPLC. (Supelco activated C18 reverse-phase column, 3% methanol mobile phase) . Yield (9 g, 85%); MS (FAB): m/e 982 (MH⁺).

### (g) Gadolinium complex of N,N'-bis[1,4,7-tris-(carboxymethyl)-1,4,7,10-tetraazacyclododecan-10-yl-methylcarbonyl]-N,N'-bis(2,3-dihydroxypropyl)-ethylenediamine

The dimeric chelant of Example 7(f) (10 g, 10.2 mmol) was dissolved in H₂O (175 mL). Gadolinium triacetate (5.45 g, 0.016 mol, 80% of the theoretical stoichiometric amount) was added and the pH was adjusted to 7 using NH₄OH. The reaction mixture was stirred at 50°C for 2 hours. Additional Gadolinium triacetate was added in 5 mol% increments until a xylenol orange test for gadolinium was positive. The reaction was stirred for an additional 24 hours and the xylenol orange test repeated, giving a positive result. Purification of the title product was achieved by preparative HPLC (Supelco activated C18 reverse-phase column, 100% H₂O mobile phase) to yield (500 mg, 5%) MS (FAB): m/e 1290 (MH⁺) Anal. calculated for C₄₀H₆₆Gd₂N₁₀O₁₈^{w}3 H₂O: C, 35.76; H, 5.4; Gd, 23.36; N, 10.43; found C, 35.3; H, 5.59; Gd, 23.17; N, 10.9.

### Example 8

### Synthesis of N,N'-bis[1,4,7-tris-(carboxymethyl)-1,4,7,10-tetraazacyclododecan-10-yl-methylcarbonyl]-N,N'-bis(2-hydroxyethyl)ethylenediamine and the gadolinium complex thereof.

### (a) bis(BOC)-bis(2-hydroxyethyl)ethylene diamine

N,N'-bis(hydroxyethyl)ethylene diamine (2 g; 13.5 mmol) was dissolved in methanol and the solution was treated with di-t-butyl dicarbonate (6.0 g; 27.5 mmol). The solution was stirred at ambient temperature for 9 hours under nitrogen and concentrated. The residue was washed with petroleum ether and dried under vacuum to obtain the title product as a colorless solid (4.48 g; 95.3 %). ¹H NMR (CDCl₃): δ 1.42 (s, 18H), 3.44-3.35 (m, 8H), 3.44 (s), 3.7 (m, 4H), 4.89 (br s, 2H).

### (b) Bis (BOC)-bis(2-benzyloxyethyl)ethylene diamine

An 80 % mineral oil suspension of sodium hydride (0.75 g; 25 mmol) was washed with tetrahydrofuran under a blanket of nitrogen and re-suspended in tetrahydrofuran (25 mL). Benzyl bromide (20 mL; 169 mmol) and the diamine of Example 8(a) (4.37 g; 12.54 mmol) were added in succession. Following a vigorous initial reaction, the suspension was stirred overnight at ambient temperature under nitrogen. The solution was concentrated under vacuum and the excess benzyl bromide was distilled off under vauum at 40°C and the residue containing approximately 10 % benzyl bromide was dried under vacuum to yield the title product as a yellow solid (7.18 g, 108 %). ¹H NMR (CDCl₃) : δ 1.39 (s, 9H), 1.42 (s, 9H), 3.38-3.66 (m, 10H), 4.49 (s, 4H), 7.29 (m, 10H).

### (c) Bis(2-benzyloxyethyl)ethylenediamine

The diamine of Example 8(b) (7.18 g) was dissolved in CH₂Cl₂ (40 mL) and cooled to 0°C. Trifluoroacetic acid (35 mL) was added and the solution was stirred at ambient temperature for 2 hours. After concentration, the title product was washed with petroleum ether and dried under vacuum. Yield : 7.2 g (93 %). ¹H NMR (CDCl₃) : δ 7.27 (m, 10H), 4.46 (s, 4H), 3.65 (t, 4H), 3.44 (s, 4H), 3.11 (t, 4H).

### (d) Bis(chloroacetyl)-bis(2-benzyloxyethyl)-ethylenediamine

A solution of the compound of Example 8(c) (6.81g; 20.75 mmol) and triethylamine (5.0 mL, 41.5 mmol) in CH₂Cl₂ (250 mL) was cooled to 0°C under nitrogen and chloroacetyl chloride (3.3 mL, 41.5 mmol) was added dropwise with stirring. The solution was stirred at ambient temperature for 24 hours and washed with water (7 x 20 mL), dried (MgSO₄), and concentrated. The crude title product was purified by column chromatography on silica gel (ether eluent) to give 2.94 g (29 %). ¹H NMR (CDCl₃) : δ 7.28 (m, 10 H), 4.44 (m, 4 H ), 4.21 (d, 4 H), 3.65-3.35 (s); 3.35 (m, 12 H).

### (e) N,N'-Bis[1,4,7-tris-(tert-butoxycarbonylmethyl)-1,4,7,10-tetraazacyclododecan-10-yl-methylcarbonyl)bis(2-benzyloxyethyl)ethylenediamine

A solution of the diamine of Example 8(d) (2.94 g, 6.1 mmol), the hydrobromide salt of Example 1(b) (7.27 g, 12.2 mmol), sodium iodide (0.9 g, 6.1 mmol), and tetramethylguanidine (2.3 mL, 18.3 mmol) in acetonitrile (130 mL) was heated at 60°C under nitrogen for 19 hours. The solvent was removed under vacuum and the residue was redissolved in CH₂Cl₂ (150 mL), washed with water (100 mL), 1 M Na₂CO₃ (2 x 100 mL) and water (100 mL). The organic layer was extracted with 1 M HCl (4 x 100 mL) and water (100 mL). The combined aqueous layer was basified with solid Na₂CO₃ and extracted with CH₂Cl₂ (4 x 100 mL). The organic layer was dried (Na₂SO₄) and concentrated. The crude title product (11 g, 62 %) was obtained as a brown solid. ¹H NMR (CDCl₃): δ 7.28 (m, 10 H), 4.45 (m, 8 H), 3.89-2.58 (m, 60 H), 1.45 (s, 18 H), 1.32 (s, 32 H).

### (f) N,N'-Bis[1,4,7-tris-(carboxymethyl)-1,4,7,10-tetraazacyclododecan-10-yl-methylcarbonyl]bis(benzyloxyethyl)ethylene diamine

The crude dimer of Example 8(e) (11 g) was dissolved in CH₂Cl₂ (50 mL) and treated with trifluoroacetic acid (40 mL). The mixture was stirred at ambient temperature for 1 hour and concentrated. This process was repeated seven times. After the final deprotection the product was chased with CH₂Cl₂ (3 x 15 mL) and water (3 x 15 mL) and purified by ion-exchange chromatography on BioRad AG1 X-8 resin (100-200 mesh, acetate form), eluting with 0.1 M acetic acid. Further purification was effected by precipitation of the product from methanol by the addition of acetone, when the title product separated as a yellow solid (4.08 g; 47 %). ¹H NMR (D₂O) : δ 7.15 (s, 10 H), 4.30 (m, 4 H), 3.60 (s), 3.60-2.87 (m, 60 H),

### (g) N,N'-Bis[1,4,7-tris-(carboxymethyl)-1,4,7,10-tetraazacyclododecan-10-yl-methylcarbonyl]bis(2-hydroxyethyl) ethylenediamine

The dimer of Example 8(f) (4.05 g; 3.68 mmol) was dissolved in a mixture of water (25 mL) and glacial acetic acid (15 mL) and treated with Pearlman's catalyst (2 g) and the mixture was hydrogenated in a Parr apparatus at 52 psi until the hydrogen uptake ceased. The solution was then filtered and concentrated. The residue was dissolved in methanol (50 mL) and precipitated with acetone addition (100 mL). This process was repeated again and the title product, a pale yellow solid was dried under vacuum. Yield : 3.07g (88.6 %). ¹H NMR (D₂O) : δ 3.59-2.93 (m) . ¹³C NMR (D₂O) : δ 177.05; 171.74; 171.4; 169.87; 169.52; 58.52; 58.35; 56.14; 55.45; 55.28; 55.04; 52.26; 52.36; 50.97; 50.38; 49.81; 48.85; 48.59; 47.84; 44.33; 43.68; 42.97; 38.68; 37.96; 29.85.

### (h) Bisgadolinium complex of N,N'-bis[1,4,7-tris-(carboxymethyl)-1,4,7,10-tetraazacyclododecan-10-yl-methylcarbonyl]bis(2-hydroxyethyl)-ethylenediamine

The dimeric chelant of Example 8(g) (3.0 g, 3.26 mmol) was dissolved in water (30 mL) and gadolinium acetate (2.38 g, 5.86 mmol) was added. The solution was stirred at ambient temperature for 2 hours and at 40°C for 3 hours. It was then concentrated , chased with water and redissolved in water. The solution gave a positive xylenol orange test. Addition of the ligand (in 1 weight % increments) followed by heating at 40°C for 1 hour was continued until the solution gave a negative xylenol orange test. The solution was then concentrated and chased with water (2 x 20 mL). Further purification by precipitation from methanol/acetone solvent system (x 6) and passage through BioRad AG1-X8 resin (acetate form) yielded the title product as a yellow solid (3.13 g, 78 %). MS (FAB) : m/e: 1230 (MH⁺). Final purification for toxicity studies was effected by semi-preparative HPLC on a C18 column. Elemental analysis; calculated for C₃₈H₆₂ Gd₂N₁₀O₁₆•13.1 H₂O:
C 31.15 %, H 6.07 %, Gd 21.46 %, N 9.56 %; found: C 31.27 %, H 5.61 %, Gd 21.0 % N 9.56 %.

### Example 9

### Synthesis of N,N'-bis[1,4,7-tris-(carboxymethyl)-1,4,7,10-tetraazacyclododecan-10-yl-methylcarbonyl]-1-(N-methylglucaminecarbonyl) - ethylenediamine and bisgadolinium complex thereof

### (a) N-BOC-N-methylglucamine

N-methylglucamine (4.0 g; 20.49 mmol) and di-t-butyl dicarbonate (4.48 g; 20.51 mmol) were dissolved in methanol (40 mL) and the solution was stirred under nitrogen for 24 hours. Concentration and washing with petroleum ether yielded the title product as a colorless solid (6.48 g, 100 %). ¹H NMR (D₂O) : 6 3.59-3.38 (m, 6 H), 3.1 (s, 2 H), 2.67 (br, 3 H), 1.21 (s, 9 H).

### (b) N-BOC-N-methyl-pentakis-(O-benzyl)glucamine

An 80 % suspension of sodium hydride (1.5 g, 50 mmol) was washed with tetrahydrofuran (20 mL) under nitrogen.

Benzyl bromide (17.8 mL, 150 mmol) and tetrahydrofuran (30 mL) were added followed by N-BOC-N-methylglucamine (Example 9(a), 2.95 g, 10 mmol). The mixture was stirred at ambient temperature under nitrogen overnight. The reaction was quenched by the gradual addition of water (30 mL) with stirring. The organic layer was separated and the aqueous layer extracted with CH₂Cl₂ (2 x 30 mL). The combined organic extract was washed with water, dried (MgSO₄) and concentrated to obtain a yellow oil from which the excess benzyl bromide was distilled off under vacuum at a bath temperature of 100°C. The crude title product was obtained as a sticky yellow solid (7 g; 94 %). ¹H NMR (CDCl₃) : δ 7.38 (m, 25 H), 4.79-4.44 (m, 10 H), 4.04-3.76 (m, 6 H), 3.49 (m, 2 H), 2.96 (s), 2.78 (d, 3 H), 1.48-1.44 (d, 9 H). ¹³C NMR (CDCl₃) : 155.72; 138.53 (m); 127.68 (m); 79.0-76.49 (m); 73.93-69.60 (m); 49.96; 35.94; 28.41.

### (c) N-Methyl-pentakis(O-benzyl)glucamine

The crude glucamine of Example 9(b) (7.02 g, 9.43mmol) was dissolved in CH₂Cl₂ (25 mL) and the solution was cooled in an ice bath. Trifluoroacetic acid (40 mL) was added and the mixture was stirred at ambient temperature for 2 hours. The solution was concentrated and the deprotection process was repeated again. The solution was chased with CH₂Cl₂ (2 x 15 mL), washed with saturated NaHCO₃ (30 mL), water (30 mL), dried (Na₂SO₄), and concentrated to yield the title product as a brown oil which was purified by chromatography on silica gel using 5 % methanol in chloroform as the eluent. Yield : 5.55 g (91 %). ¹H NMR (CDCl₃) : δ 7.33 (m, 25 H), 4.8-4.41 (m, 10 H), 4.06-3.61 (m, 6 H) 3.03-2.71 (m, 2 H), 2.41 (s, 3 H). ¹³C NMR (CDCl₃) : δ 137.97 (m); 128.25; 78.85-69.55 (m); 50.41; 39.94.

### (d) N,N'-Bis(BOC)-2,3-diaminopropionic acid

A suspension of diaminopropionic acid hydrochloride (5.13 g, 36.5 mmol) in a mixture of ethanol (70 mL) and methanol (20 mL) was treated with triethylamine (10.2 mL, 73 mmol), diisopropylethylamine (5 mL) and di-t-butyldicarbonate (16.72 g, 76.6 mmol). The mixture was stirred at ambient temperature overnight and refluxed for 7 hours under nitrogen. The solution was filtered and concentrated. The residue was washed with petroleum ether (3 x 20 mL), treated with chloroform (150 mL) and the mixture cooled to 0°C. 1 M H₂SO₄ (150 mL) was added and the mixture was stirred at 0°C for 15 minutes. The aqueous layer was removed and extracted with chloroform (2 x 30 mL). The combined organic layers were washed with water, dried (Na₂SO₄) and concentrated to obtain the title product as a colorless solid (9.57 g; 86.3 %). ¹H NMR (CDCl₃) : δ 7.34 (br, 1 H), 6.25-5.19 (br, 2 H), 4.3 (br, 1 H), 3.52 (m, 2 H), 1.42 (s, 18 H). ¹³C NMR (CDCl₃) : δ 173.32; 54.69; 42.22; 28.29.

### (e) N,N'-Bis(BOC)-(N-methyl-pentakis-O-benzylglucaminecarbonyl)-ethylenediamine

A mixture of the propionic acid of Example 9(d) (2.24 g, 7.37 mmol), dicyclohexylcarbodiiimide (1.52 g, 7.37 mmol), the glucamine of Example 9(c) (4.76 mg, 7.37 mmol) and dimethylaminopyridine (0.09 mg, 0.74 mmol) in CH₂Cl₂ (50 mL) was stirred at ambient temperature under nitrogen overnight. The solution was filtered, washed with water, dried (MgSO₄) and concentrated to give a brown oil. Purification by column chromatography on silica gel (chloroform eluent) yielded the title product (6.19 g; 90 %). ¹H NMR (CDCl₃) : δ 7.32 (m, 25 H), 4.81-4.65 (m, 10 H), 4.04-3.75 (m, 4 H), 1.45 (d, 18 H). ¹³C NMR (CDCl₃) : δ 170.17, 169.92, 155.8, 155.2, 138.21, 127.76, 79.46-76.49 (m), 74.30-69.21 (m), 50.78-48.65 (m), 42.59, 36.31, 28.15, 28.1, 25.46, 24.81.

### (f) N-methyl-pentakis(O-benzyl)qlucaminecarbonylethylenediamine

The ethylenediamine of Example 9(e) (5g) was dissolved in CH₂Cl₂ (60 mL) and the solution was cooled to 0°C. Trifluoroacetic acid (60 mL) was added and the solution was stirred at ambient temperature for 2 hours. The solution was concentrated and the process was repeated again. After concentration the residue was chased with CH₂Cl₂ (3 x 15 mL), dissolved in CHCl₃, and washed with saturated NaHCO₃ (2 x 30 mL) and water and dried (MgSO₄). The title product was obtained as a brown oil after concentration (4.72 g). ¹H NMR (CDCl₃) : δ 7.3 (m, 25 H), 4.76-4.25 (m, 11 H), 3.96-3.41 (m, 10 H), 2.94-2.52 (m, 3 H). ¹³C NMR (CDCl₃) : δ 138.62, 127.7, 78.89-76.5 (m), 74.63-69.55 (m), 53.62-46.0 (m), 39.62-24.90. MS (FAB) : m/e: 732.6 (MH⁺).

### (g) N-Methyl-pentakis(O-benzyl)glucaminecarbonylethylenediamine-bis(chloroacetamide)

The ethylenediamine of Example 9(f) (3.9 g, 5.33 mmol) and triethylamine (1.5 mL, 10.7 mmol) were dissolved in chloroform (50 mL) and the solution was cooled to 0°C under nitrogen. Chloroacetyl chloride (1.2 g, 10.6 mmol) was added slowly. After the addition was complete the solution was warmed to ambient temperature and stirred overnight. The solution was washed with water (3 x 20 mL), dried (MgSO₄) and concentrated. The residue was chromatographed on silica gel, eluting with methanol/chloroform solvent mixture (0-5 %) to yield the title product 3.82 g (81 %). ¹H NMR (CDCl₃) : δ 7.30 (m, 25 H), 4.78-4.40 (m, 10 H), 4.30 (m, 1 H), 4.01-3.19 (m, 14 H), 2.94-2.51 (m, 3 H) . ¹³C NMR (CDCl₃) : δ 168.85-165.55 (m), 137.98-137.5 (m), 127.86-126.74 (m), 78.29-75.55 (m), 74.09-71.38 (m), 68.94-68.72 (m), 49.08-48.64 (m), 42.14-40.99 (m), 36.5; 35.8, 33.76-33.1, 25.07, 24.41. MS (FAB): m/e 884.4 (MH⁺).

### (h) N,N'-Bis[1,4,7-tris-(tert-butoxycarbonylmethyl)-1,4,7,10-tetraazacyclododecan-10-yl-methylcarbonyl]-N-methyl-pentakis (O-benzyl)glucamine-carbonyethylenediamine

The hydrobromide salt of Example 1(b) (5.12 g, 8.6 mmol) and the bis(chloroacetamide) of Example 9(g) (3.8 g, 4.3 mmol) were dissolved in CH₃CN (100 mL) and tetramethylguanidine (1.62 mL, 12.9 mmol) was addded. The solution was heated at 60°C under nitrogen for 25 hours. After concentration, the residue was taken up in chloroform and washed with water and dried (MgSO₄). Following concentration, the crude title product was obtained as a viscous yellow material (9.08 g). ¹H NMR (CDCl₃) : δ 7.22 (m, 25 H), 4.70-4.32 (m, 11 H), 4.00-3.67 (m, 6 H), 3.38-2.49 (m, 55 H), 1.39-1.35 (m, 54 H). ¹³C NMR (CDCl₃) : δ 174.27 -169.56 (m), 138.5-136.6 (m), 132.49-127.11 (m), 81.06-80.16 (m), 78.62-76.49 (m), 73.62-69.37(m), 57.62-47.28 (m), 36.9; 27.97. MS (FAB): m/e 1842.1 (MH⁺).

### (i) N,N'-Bis[1,4,7-tris-(carboxymethyl)-1,4,7,10-tetraazacyclododecan-10-yl-methylcarbonyl]-N-methylpentakis(O-benzyl)glucamine-carbonylethylenediamine

The dimer of Example 9(h) (7.9 g, 4.29 mmol) was dissolved in dichloromethane (80 mL) and cooled to 0°C. Trifluoroacetic acid (80 mL) was added and the solution was stirred at ambient temperature for 1 hour. The solution was concentrated and the process was repeated nine times. After the final deprotection, the solution was concentrated and chased with CH₂Cl₂ (4 x 20 mL) and water (4 x 20 mL). Drying under vacuum yielded the crude title product (10.9 g). ¹H NMR (D₂O) : δ 6.53 (br, 25 H), 4.08-2.37 (m, 72 H). ¹³C NMR (D₂O) : δ 126.33, 52.33, 51.05, 50.13, 47.27, 45.68, 40.27. MS (FAB) : m/e 1504.5 (MH⁺).

### (j) N,N'-Bis[1,4,7-tris-(carboxymethyl)-1,4,7,10-tetraazacyclododecan-10-yl-methylcarbonyl]-1-(N-methylglucaminecarbonyl)-ethylenediamine

A solution of the dimer of Example 9(i) (0.86 g, 0.57 mol) in water (20 mL) was treated with glacial acetic acid (5 mL) and Pearlman's catalyst (0.5 g). The mixture was hydrogenated in a Parr hydrogenation apparatus with hydrogen gas at a pressure of 53 psi until no further uptake of hydrogen was observed. The solution was filtered, concentrated and chased with water. The residue was purified by ion exchange chromagraphy using BioRad AG1-X8 resin (100-200 mesh, acetate form) using acetic acid as the eluent (0.05-0.2 M) to give 0.42 g (70 %) of the title product as a pale yellow solid. ¹H NMR (D₂O) : δ 3.8 -2.66 (m). ¹³C NMR (D₂O): δ 175.17, 179.46, 172.17-170.77, 72.22-70.61, 63.31, 56.87-48.77, 40.59-35.18. MS (FAB): m/e 1054.6 (MH⁺).

### (k) Synthesis of Bisgadolinium complex of N,N'-bis[1,4,7-tris-(carboxy-methyl)-1,4,7,10-tetraazacyclododecan-10-yl-methylcarbonyl]-1-(N-methylglucaminecarbonyl)ethylenediamine

To a solution of the dimeric chelant of Example 9(j) (0.94 g, 0.89 mmol) in water (25 mL) was added gadolinium acetate (0.47 g) and the solution was stirred at 40°C overnight. It was concentrated and the solution was repeatedly chased with water until the pH was to 5.2. The xylenol orange test for free gadolinium was negative. Further quantities of gadolinium acetate were added in 1 weight % increments and the solution heated at 40°C for 1 hour until the reaction mixture showed a postive xylenol orange test for gadolinium. The solution was filtered, concentrated, and chased with water. The crude title product was subjected to purification by semi-preparative HPLC on a C18 column using 2 % methanol in water, followed by precipitation from a methanol solution with acetone to give 0.78 g (52 %). MS (FAB): m/e 1364.3 (MH⁺).

### Example 10

### Synthesis of bis[1,4,7-tris-(carboxymethyl)-1,4,7,10-tetraazacyclododecan-10-yl]-2-oxo-3-aza-pentane and the gadolinium and dysprosium complexes thereof

### (a) 3-Aza-4-oxo-1,5-dibromopentane

Bromoethylamine hydrobromide (6.15 g, 30 mmol) was suspended in chloroform (40 mL) and treated with diisopropylethylamine (5.2 mL, 60 mmol). The solution was cooled in a dry ice acetone bath and a solution of bromoacetyl bromide (2.6 mL, 30 mmol) in chloroform (10 mL) was added dropwise under nitrogen. After the addition was complete, the solution was warmed to ambient temperature and stirred overnight. The solution was washed successively with water (2 x 30 mL), 1 M acetic acid (2 x 30 mL), water (30 mL), 1 M NaOH (2 x 30 mL) and water (2 x 30 mL). Drying over anhydrous Na₂SO₄ followed by concentration yielded the crude product as a colorless solid (4.94 g; 66 %). Recrystallization from chloroform yielded the pure title product (1.33 g, 18.1 %). ¹H NMR (CDCl₃) : δ 6.84 (br, 1 H), 3.91 (s, 2 H), 3.72 (q, 2 H), 3.5 (t, 2 H). ¹³C NMR (CDCl₃) : δ 166.08, 41.54, 31.11, 28.67.

### (b) Bis [1,4,7-tris-(tert-butoxycarbonylmethyl)-1,4,7,10-tetraazacyclododecan-10-yl]-2-oxo-3-azapentane

3-Aza-4-oxo-1,5-dibromopentane (Example 10(a), 1.28 g, 5.22 mmol), the hydrobromide salt of Example 1(b) (6.22 g, 10.44 mmol) and tetramethylguanidine (2 mL, 15.66 mmol) were dissolved in acetonitrile (125 mL) and the solution was heated at 60°C for 48 hours under nitrogen. The solution was concentrated, the residue was dissolved in chloroform and washed with water. It was dried over anhydrous Na₂SO₄ and concentrated to yield a brown oil. Petroleum ether extraction followed by concentration of the extract yielded the crude title product (6.26 g). ¹H NMR (CDCl₃): 8.8 (br, 1 H), 3.46-2.38 (m, 50 H), 1.28 (s, 54 H). ¹³C NMR (CDCl₃) : δ 172.2-169.84, 80.47, 61.83-47.37, 27.94, 27.9. MS (FAB): m/e 1112.9 (MH⁺).

### (c) Bis[1,4,7-tris-[carboxymethyl)-1,4,7,10-tetraazacyclododecan-10-yl]-2-oxo-3-aza-pentane

To a solution of the dimer of Example 10(b) (6.25 g) in CH₂Cl₂ (60 mL) cooled in an ice bath, is added trifluoroacetic acid (60 mL) and the mixture stirred at ambient temperature for 1 hour. The solution is concentrated and the process is repeated nine times.

After the final deprotection, the solution is concentrated and chased with CH₂Cl₂ (3 x 20 mL) and water (3 x 20 mL). The residue is passed through BioRad AG1 X-8 ion exchange resin (100-200 mesh, acetate form) and the product eluted with aqueous acetic acid (0.05-0.1 M acetic acid).

### (d) Bisgadolinium complex of bis[1,4,7-tris-(carboxymethyl)-1,4,7,10-tetraazacyclododecan-10-yl]-2-oxo-3-aza-pentane

The dimeric chelant of Example 10(c) is dissolved in water and gadolinium acetate is added. The solution is stirred at 40°C for 2 hours. The pH of the solution is raised from 3 to 5 by adding 0.1 M ammonium hydroxide followed by chasing with water. Addition of gadolinium acetate in 1 wt. % increments and heating at 40°C is continued until a positive xylenol orange test is observed. The solution is then filtered and concentrated. The residue is chased with water and dried under vacuum to obtain the title product.

### (e) Bis dysprosium complex of bis[1,4,7-tris-(carboxymethyl)-1,4,7,10-tetraazacyclododecan-10-yl]-2-oxo-3-aza-pentane

The dysprosium complex is prepared analogously to Example 10(d) using the dimeric chelant of Example 10(c) and a soluble dysprosium(III) salt.

### Example 11 - Experimental Results

Gadolinium complexes of a series of dimeric substituted tetraazacyclododecane macrocycles (Table 1) have been synthesized. Their physicochemical properties were studied to evaluate their utility as extracellular fluid MRI contrast agents. The results are presented here.

### Experimental

The DO3A bis(amide) dimer ligands 1-11 shown in Table 1 were prepared by the coupling reaction of D03A-tri-t-butyl ester with bis(chloroacetamides) of the appropriate diamines, followed by the deprotection of the t-butyl ester groups. The gadolinium complexes were prepared by the reaction of the ligands with Gd(OAc)₃. Relaxivities were measured in water and in serum (in selected cases) at 40°C and 20 MHz. Viscosities and osmolalities were measured at concentrations listed in Table 2.

### Conclusion

The favourable physicochemical profiles of the dimeric gadolinium chelates presented here, which include relaxivity, viscosity and osmolality suggest their potential use as new extracellular fluid MRI contrast agents.

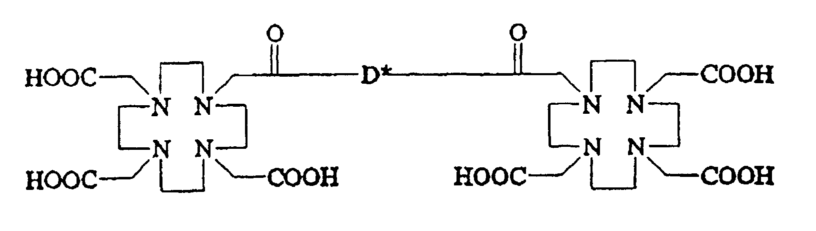

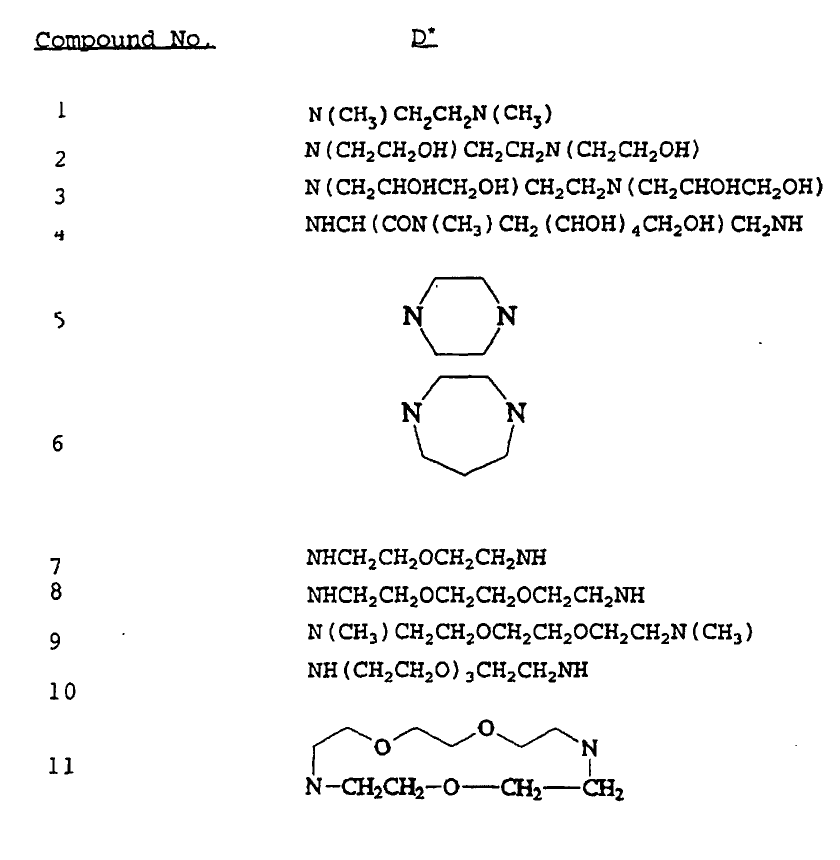

**Table 2 -**

| **Physicochemical properties of complexes** | | | | | | |
|---|---|---|---|---|---|---|
| Complex | Relaxivity^{a} (mM⁻¹ sec⁻¹ Gd⁻¹) | | Osmolality^{b} (mmol/kg) | | Viscosity^{c} (cPs) | |
| | r₁ | r₂ | | | 25°C | 37°C |
| 1 | 5.6 | 5.5 | 323 | (200) | 2.0 | 1.4 |
| 2 | 5.2 | 4.9 | 555 | (200) | 2.1 | 1.5 |
| 3 | 5.5 | 6.2 | 345 | (275) | .^{d} | .^{d} |
| 4 | 6.1 | 6.9 | 1083 | (384) | .^{d} | .^{d} |
| 5 | 5.8 | 6.6 | 581 | (250) | 3.0 | 2.0 |
| 6 | 5.1 | 5.9 | 474 | (250) | 2.6 | 1.8 |
| 7 | 4.7 | 4.8 | 653 | (314) | 2.8 | 2.1 |
| 8 | 4.7 | 5.7 | 752 | (308) | 3.3 | 2.4 |
| 9 | 5.0 | 6.5 | 635 | (278) | 2.7 | 2.0 |
| 10 | 4.4 | 5.3 | 793 | (281) | 3.4 | 2.6 |
| 11 | 5.7 | 7.1 | 971 | (257) | 2.9 | 2.1 |

| | | | | | | |
|---|---|---|---|---|---|---|
| ^{a} in water at 40°C and 20 MHz | | | | | | |
| ^{b} concentrations (mM) in parentheses | | | | | | |
| ^{c} same concentrations as in osmolality unless otherwise stated | | | | | | |
| ^{d} not measured | | | | | | |

## Claims

1. Dichelants of formula V 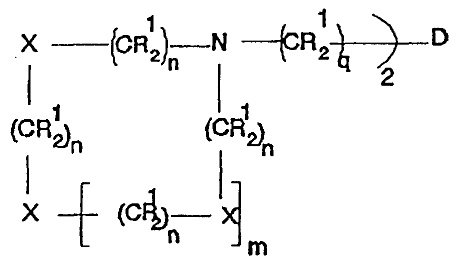 and salts or metal chelates thereof,
(wherein each X which may be the same or different is NZ, O or S, at least two Xs being NZ;
each Z is a group R¹ or a group CR¹₂Y, at least one Z on each macrocyclic ring being a group CR¹₂Y;
each Y is a group CO₂H, PO₃H, SO₃H, CONR¹₂, CON(OR¹)R¹, CNS or CONR¹NR¹₂;
m is 0 or 1 or 2;
each n is 2 or 3;
q is 1 or 2;
each R¹ which may be the same or different is a hydrogen atom or a C₁₋₆ alkyl group optionally substituted by one or more hydroxy and/or C₁₋₆ alkoxy groups;
and D is a bridging group, other than an unsubstituted carbonylaminoethylaminocarbonyl group, of formula
-CO-X²-L¹ (̵X²-CO )̵ₚ
having a molecular weight of less than 1000,
containing one or more ether oxygens,
where p is 0 or 1,
X² is O or NR²,
R² is a hydrogen atom or a hydroxy, OR¹ or NR¹₂ group or a C₁₋₆ alkyl group optionally interrupted by oxygen, sulphur or nitrogen atoms or by carbonyl or aryl groups and optionally substituted by hydroxyl, amine or aryl groups, or R² contains a functional group for attachment to a biomolecule or macromolecule, or two R² groups together form a bridging linker group, and L¹ which provides a chain or at least two atoms linking two X² groups or at least one atom linking an X² group and a (CR¹₂)_{q} moiety (wherein R¹ and q are as defined above), is a straight chain, branched or cyclic alkylene group or a combination of such groups interrupted by oxygen, optionally substituted and optionally being interrupted by sulphur or nitrogen atoms or by aryl or carbonyl groups.

2. Compounds as claimed in claim 1 wherein each macrocyclic 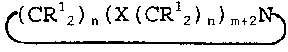 ring has 9 to 14 ring atoms.

3. Compounds as claimed in either of claims 1 and 2 wherein in each said macrocyclic ring each n is 2.

4. Compounds as claimed in any one of claims 1 to 3 wherein bridging group D has a molecular weight of less than 500.

5. Compounds as claimed in any one of claims 1 to 4 wherein each q is 1.

6. Compounds as claimed in any one of claims 1 to 5 wherein each m is 1.

7. Compounds as claimed in any one of claims 1 to 6 wherein each y is a group COOH or COO^{⊖}.

8. Compounds as claimed in any one of claims 1 to 7 wherein D is of a formula selected from the group consisting of 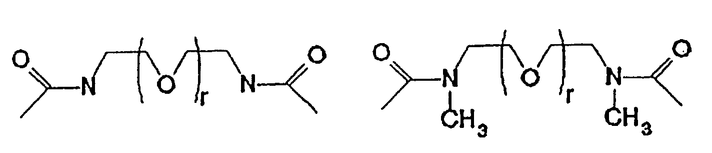 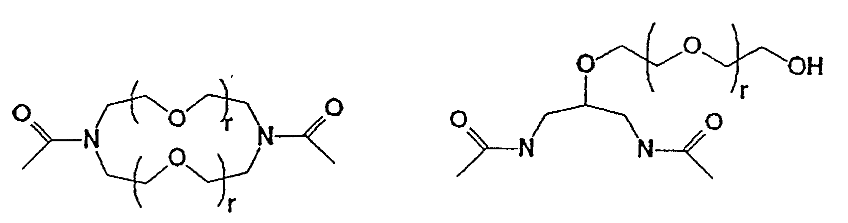 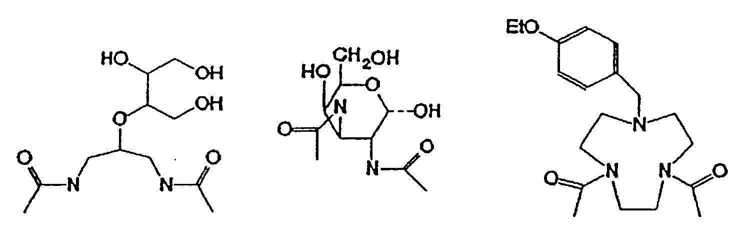 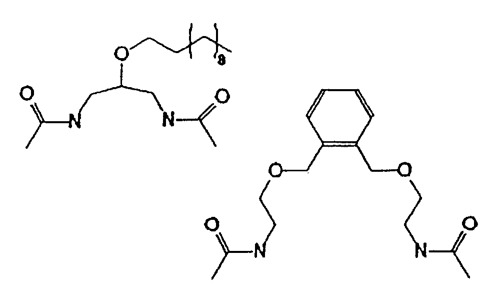 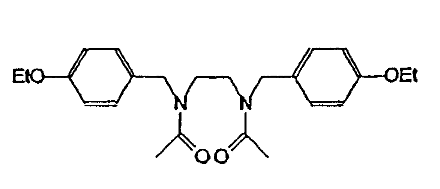 where r is an integer having a value of 1 to 6, and s is an integer having a value of 1 to 20.

9. Compounds as claimed in any one of claims 1 to 7 wherein D is of formula COOCH₂CH₂OCH₂CH₂NHCH₂CH₂OCH₂CH₂OCO.

10. Compounds as claimed in any one of claims 1 to 7 wherein D is of formula CONHCH₂CH₂OCH₂CH₂OCH₂CH₂NHCO.

11. Compounds as claimed in claim 1 being chelants of formula VII
M-CH₂CO)₂-D' (VII)
where M is a nitrogen attached triaza, tetraaza, triazaoxa or triazathia-cycloalkane having at least one and preferably two ring nitrogens substituted by CH₂COOH groups and having any remaining ring nitrogen substituted by a group R³;
R³ is a hydrogen atom, or a C₁₋₆ alkyl group optionally mono or polysubstituted by hydroxyl or C₁₋₆ alkoxy groups and optionally interrupted by arylene or substituted arylene groups; and
CO-D'-CO is a bridging group as defined for D in claim 1 and salts and metal chelates thereof.

12. A process for the preparation of compounds as claimed in claim 1 said process comprising at least one of the following steps:
(a) reacting a compound of formula V wherein at least one group X is a group NH, with a compound of formula VIII
Lv-R⁴ (VIII)
(where Lv is a displaceable leaving group and R⁴ is a group CR¹₂Y or group R¹ other than hydrogen);
(b) reacting compounds of formulae X and/or XI 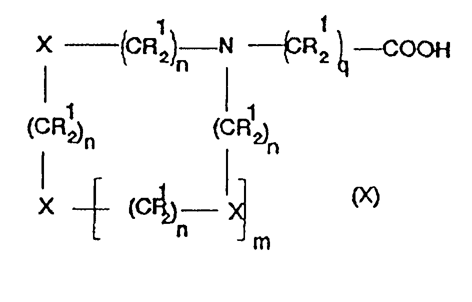 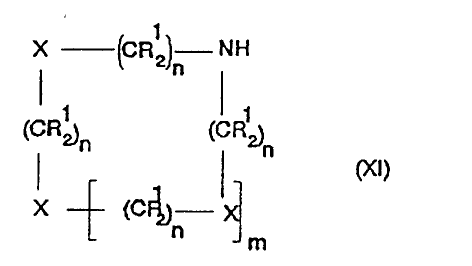 (wherein X, R¹, n, q and m are as defined in claim 1) or an activated derivative thereof with a linker molecule of formula IX
Lv(CO-X² )̵L¹- (X²-CO)ₜLv (IX)
or formula IXa
Lv-(CR¹ ₂)_{q}-(CO-X² )̵L¹-(X²-CO)ₜ-(CR¹ ₂)_{q}-Lv (IXa)
(wherein R¹, q, X² and L¹ are as defined in claim 1, t is 0 or 1, and each Lv is a displaceable leaving group, one optionally being protected prior to conjugation of the second compound of formula X or XI);
(c) converting a compound of formula X into a corresponding acid chloride and reacting with a suitable diamine;
(d) metallating or transmetallating a compound of formula V or a chelate thereof;
(e) converting a compound of formula V or a chelate thereof into a base or acid addition salt thereof or converting a salt into the free acid or base; and
(f) performing at least one of steps (a) to (d) above using reagents with protected functional groups and subsequently removing the protecting groups.

13. A process for the preparation of a metal chelate of which process comprises admixing in a solvent a compound of formula V as defined in claim 1 or a salt or chelate thereof together with an at least sparingly soluble compound of said metal.

## Patentansprüche

1. Dichelatbildner der Formel V 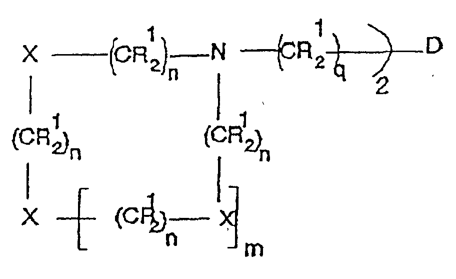 und Salze oder Metallchelate hiervon,
(worin jedes X, das gleich oder verschieden sein kann, NZ, O oder S ist, wobei mindestens zwei Xs NZ sind;
jedes Z eine Gruppe R¹ oder eine Gruppe CR¹₂Y ist, wobei mindestens ein Z an jedem makrocyclischen Ring eine Gruppe CR¹₂Y ist;
jedes Y eine Gruppe CO₂H, PO₃H, SO₃H, CONR¹₂, CON(OR¹)R¹, CNS oder CONR¹NR¹₂ ist;
m 0 oder 1 oder 2 ist;
jedes n 2 oder 3 ist;
q 1 oder 2 ist;
jedes R¹, das gleich oder verschieden sein kann, ein Wasserstoffatom oder eine C₁₋₆-Alkylgruppe ist, wahlweise substituiert durch eine oder mehrere Hydroxyund/oder C₁₋₆-Alkoxygruppen;
und D eine von einer unsubstituierten Carbonylaminoethylaminocarbonylgruppe verschiedene Brückengruppe der Formel
-CO-X²-L¹ -(X²-CO )ₚ
mit einem Molekulargewicht von weniger als 1.000 ist,
enthaltend einen oder mehrere Ethersauerstoffatome,
worin p 0 oder 1 ist,
X² O oder NR² ist,
R² ein Wasserstoffatom oder eine Hydroxy-, OR¹- oder NR¹₂-Gruppe oder eine C₁₋₆-Alkylgruppe ist, wahlweise unterbrochen durch Sauerstoff-, Schwefel- oder Stickstoffatome oder durch Carbonyl- oder Arylgruppen, und wahlweises substituiert durch Hydroxyl-, Amin- oder Arylgruppen, oder R² enthält eine funktionelle Gruppe zur Bindung an ein Biomolekül oder Makromolekül, oder zwei R²-Gruppen bilden zusammen eine Brückenlinkergruppe, und L¹, das eine Kette oder mindestens zwei Atome, welche zwei X²-Gruppen verbinden, oder mindestens ein Atom, das eine X²-Gruppe und eine (CR¹₂)_{q}-Einheit (worin R¹ und q wie oben definiert sind) verbindet, vorsieht, eine geradkettige, verzweigte oder cyclische Alkylengruppe oder eine Kombination solcher Gruppen ist, unterbrochen durch Sauerstoff, wahlweise substituiert und wahlweise unterbrochen durch Schwefel- oder Stickstoffatome oder durch Aryl- oder Carbonylgruppen).

2. Verbindungen nach Anspruch 1, wobei jeder makrocyclische 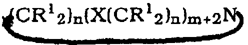 Ring 9 bis 14 Ringatome besitzt.

3. Verbindungen nach Anspruch 1 und/oder 2, wobei in jedem des makrocycllschen Rings jedes n 2 ist.

4. Verbindungen nach mindestens einem der Ansprüche 1 bis 3, wobei die Brückengruppe D ein Molekulargewicht von weniger als 500 aufweist.

5. Verbindungen nach mindestens einem der Ansprüche 1 bis 4, wobei jedes q 1 ist.

6. Verbindungen nach mindestens einem der Ansprüche 1 bis 5, wobei jedes m 1 ist.

7. Verbindungen nach mindestens einem der Ansprüche 1 bis 6, wobei y eine Gruppe COOH oder COO^{⊖} ist.

8. Verbindungen nach mindestens einem der Ansprüche 1 bis 7, wobei D eine Formel aufweist, gewählt aus der Gruppe, bestehend aus 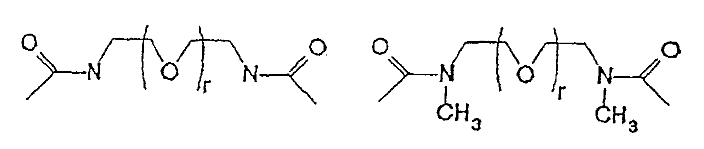 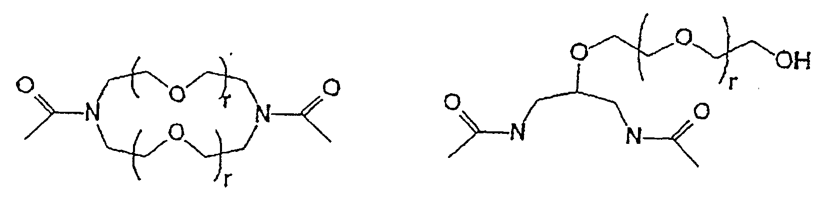 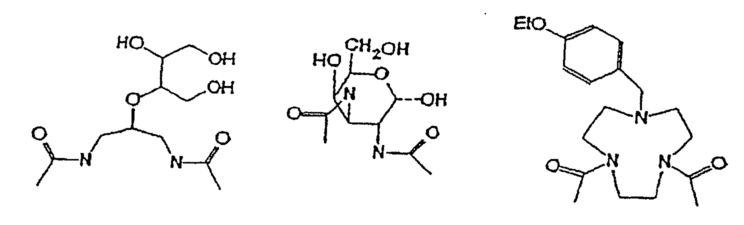 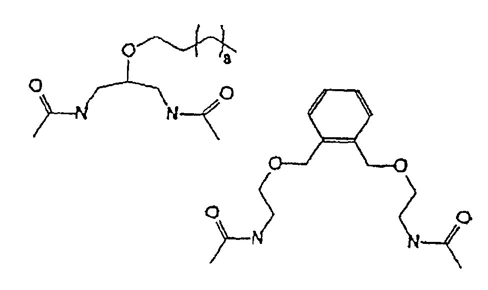 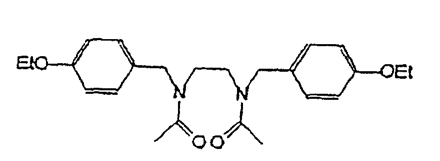 worin r eine ganze Zahl mit einem Wert von 1 bis 6, und s eine ganze Zahl mit einem Wert von 1 bis 20 ist.

9. Verbindungen nach mindestens einem der Ansprüche 1 bis 7, wobei D die Formel COOCH₂CH₂OCH₂CH₂NHCH₂CH₂OCH₂CH₂OCO besitzt.

10. Verbindungen nach mindestens einem der Ansprüche 1 bis 7, wobei D die Formel CONHCH₂CH₂OCH₂CH₂OCH₂CH₂NHCO besitzt.

11. Verbindungen nach Anspruch 1, welche Chelatbildner der Formel VII sind
M-CH₂CO)₂-D' (VII)
worin M ein Stickstoff-gebundenes Triaza-, Tetraaza-, Triazaoxa- oder Triazathla-Cycloalkan ist, bei dem mindestens ein, vorzugsweise zwei Ring-Stickstoffe substituiert sind durch CH₂COOH-Gruppen, und bei dem beliebige verbleibende Ring-Stickstoffe durch eine Gruppe R³ substituiert sind:
R³ ein Wasserstoffatom oder eine C₁₋₆-Alkylgruppe ist, wahlweise mono- oder polysubstituiert durch Hydroxyl- oder C₁₋₆-Alkoxygruppen und wahlweise unterbrochen durch Arylen- oder substituierte Arylengruppen; und
CO-D'-CO eine Brückengruppe ist, wie für D in Anspruch 1 definiert,
und Salze und Metallchelate hiervon.

12. Verfahren zur Herstellung von Verbindungen gemäß Anspruch 1, wobei das Verfahren mindestens einen der folgenden Schritte umfasst:
(a) Umsetzen einer Verbindung der Formel V, worin mindestens eine Gruppe X eine Gruppe NH ist, mit einer Verbindung der Formel VIII
Lv-R⁴ (VIII)
(worin Lv eine austauschbare Abgangsgruppe ist und R⁴ eine Gruppe CR¹₂Y oder eine von Wasserstoff verschiedene Gruppe R¹ ist);
(b) Umsetzen von Verbindungen der Formeln X und/oder XI 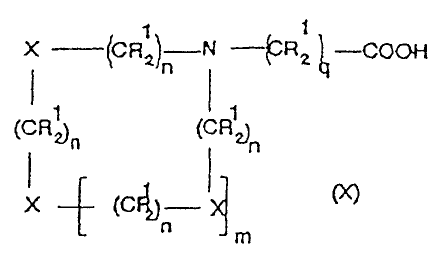 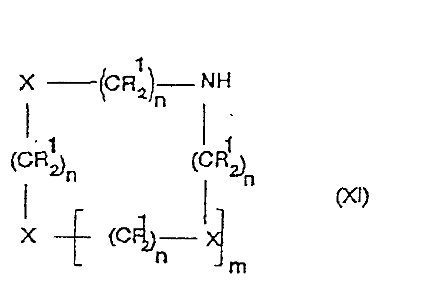 (worin X, R¹, n, q und m wie in Anspruch 1 definiert sind) oder ein aktiviertes Derivat hiervon mit einem Linkermolekül der Formel IX
Lv(CO-X² )L¹-(x²-CO)ₜLv (IX)
oder der Formel IXa
Lv-(CR¹ ₂)_{q}-(CO-X² )L¹-(X²-.CO)ₜ-(CR¹ ₂)_{q}-LV (IXa)
(worin R¹, q, X² und L¹ wie in Anspruch 1 definiert sind, t 0 oder 1 ist, und jedes Lv eine austauschbare Abgangsgruppe ist, von denen eine wahlweise vor der Konjugation der zweiten Verbindung der Formel X oder XI geschützt ist);
(c) Umwandeln einer Verbindung der Formel X in ein korrespondierendes Säurechlorid und Umsetzen mit einem geeigneten Diamin;
(d) Metallieren oder Transmetallieren einer Verbindung der Formel V oder eines Chelates hiervon;
(e) Umwandeln einer Verbindung der Formel V und eines Chelates hiervon in ein Basen- oder Säureadditionssalz hiervon oder Umwandeln eines Salzes in die freie Säure oder Base; und
(f) Durchführen mindestens eines der obigen Schritte (a) bis (d) unter Verwendung von Reagenzien mit geschützten funktionellen Gruppen und nachfolgend Entfernen der Schutzgruppen.

13. Verfahren zur Herstellung eines Metallchelates, welches Verfahren das Vermischen in einem Lösungsmittel einer Verbindung der Formel V wie in Anspruch 1 definiert oder eines Salzes oder eines Chelats hiervon, zusammen mit mindestens einer geringlöslichen Verbindung des Metalls umfasst.

## Revendications

1. Dichélateurs de formule V 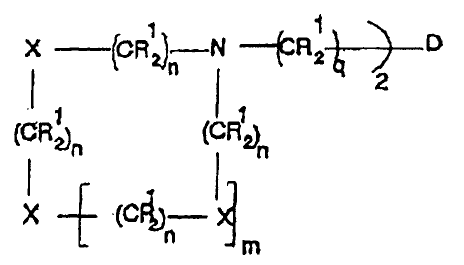 et les sels ou chélates métalliques de ceux-ci,
(dans laquelle chaque X, qui peuvent être identiques ou différents, est NZ, O ou S, au moins deux X étant NZ ;
chaque Z est un groupe R¹ ou un groupe CR¹₂Y, au moins un Z sur chaque noyau macrocyclique étant un groupe CR¹₂Y ;
chaque Y est un groupe CO₂H, PO₃H, SO₃H, CONR¹₂, CON(OR¹)R¹, CNS ou CONR¹NR¹₂ ;
m est 0 ou 1 ou 2 ;
chaque n est 2 ou 3 ;
q est 1 ou 2 ;
chaque R¹, qui peuvent être identiques ou différents, est un atome d'hydrogène ou un groupe alkyle en C₁ à C₆ éventuellement substitué par un ou plusieurs groupes hydroxy et/ou alcoxy en C₁ à C₆ ;
et D est un groupe pontant, autre qu'un groupe carbonylaminoéthylaminocarbonyle non substitué, de formule
-CO-X²-L¹ (̵X²-CO )̵ₚ
ayant un poids moléculaire inférieur à 1000,
contenant un ou plusieurs oxygènes d'éther,
où p est 0 ou 1,
X² est O ou NR²,
R² est un atome d'hydrogène ou un groupe hydroxy, OR¹ ou NR¹₂ ou un groupe alkyle en C₁ à C₆ éventuellement interrompu par des atomes d'oxygène, de soufre ou d'azote ou par des groupes carbonyles ou aryles, et éventuellement substitué par des groupes hydroxyles, amines ou aryles, ou R² contient un groupe fonctionnel pour l'attachement à une biomolécule ou macromolécule, ou deux groupes R² forment ensemble un groupe lieur pontant, et L¹, qui représente une chaîne ou au moins deux atomes liant deux groupes X² ou au moins un atome liant un groupe X² et une fraction (CR¹₂)_{q} (où R¹ et q sont tels que définis ci-dessus), est un groupe alkylène à chaîne linéaire, ramifié ou cyclique ou une combinaison de tels groupes interrompus par de l'oxygène, éventuellement substitué et éventuellement interrompu par des atomes de soufre ou d'azote ou par des groupes aryles ou carbonyles.

2. Composés selon la revendication 1, dans lesquels chaque noyau macrocyclique 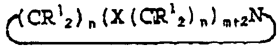 a 9 à 14 atomes dans le noyau.

3. Composés selon l'une quelconque des revendications 1 et 2, dans lesquels dans chaque noyau macrocyclique chaque n est 2.

4. Composés selon l'une quelconque des revendications 1 à 3, dans lesquels le groupe pontant D a un poids moléculaire inférieur à 500.

5. Composés selon l'une quelconque des revendications 1 à 4, dans lesquels chaque q est 1.

6. Composés selon l'une quelconque des revendications 1 à 5, dans lesquels chaque m est 1.

7. Composés selon l'une quelconque des revendications 1 à 6, dans lesquels chaque y est un groupe COOH ou COO^{⊖}.

8. Composés selon l'une quelconque des revendications 1 à 7, dans lesquels D a une formule choisie dans le groupe constitué par 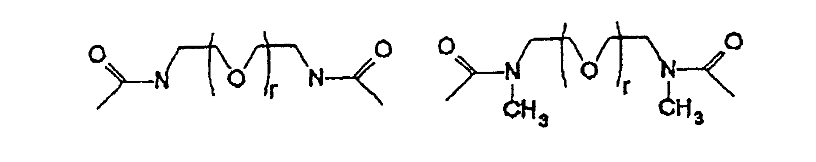 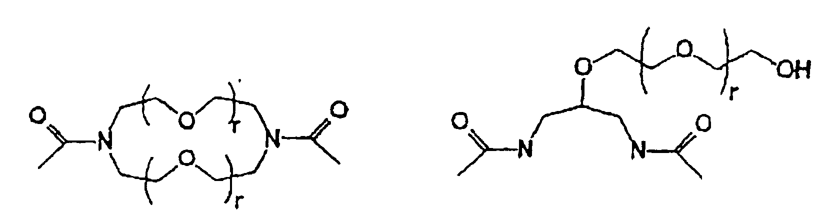 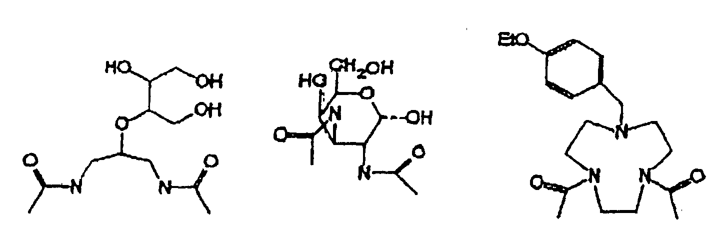 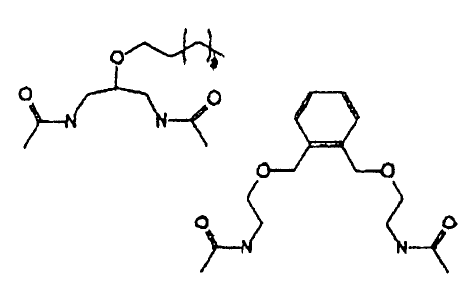 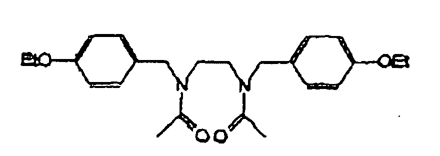 où r est un entier ayant une valeur de 1 à 6, et s est un entier ayant une valeur de 1 à 20.

9. Composés selon l'une quelconque des revendications 1 à 7, dans lesquels D a la formule COOCH₂CH₂OCH₂CH₂NHCH₂OH₂OCH₂CH₂OCO.

10. Composés selon l'une quelconque des revendications 1 à 7, dans lesquels D a la formule CONHCH₂CH₂OCH₂CH₂OCH₂CH₂NHCO.

11. Composés selon la revendication 1, qui sont des chélateurs de formule VII
M-CH₂CO)₂-D' (VII)
dans laquelle M est un azote attaché à un triaza, tétraaza, triazaoxa ou triazathia-cycloalcane ayant au moins un, et de préférence deux azotes du noyau substitués par des groupes CH₂COOH et ayant tout azote restant du noyau substitué par un groupe R³ ;
R³ est un atome d'hydrogène ou un groupe alkyle en C₁ à C₆ éventuellement mono ou polysubstitué par des groupes hydroxyles ou alcoxy en C₁ à C₆ et éventuellement interrompu par des groupes arylènes ou arylènes substitués ; et
CO-D'-CO est un groupe pontant tel que défini pour D dans la revendication 1, et les sels et chélates métalliques de ceux-ci.

12. Procédé pour la préparation de composés selon la revendication 1, ledit procédé comprenant au moins l'une des étapes suivantes :
(a) la réaction d'un composé de formule V dans laquelle au moins un groupe X est un groupe NH, avec un composé de formule VIII
Lv-R⁴ (VIII)
(où Lv est un groupe partant déplaçable et R⁴ est un groupe OR¹₂Y ou un groupe R¹ autre que l'hydrogène) ;
(b) la réaction de composés des formules X et/ou XI 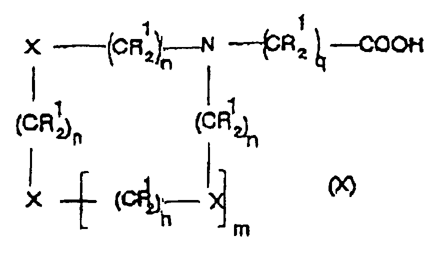 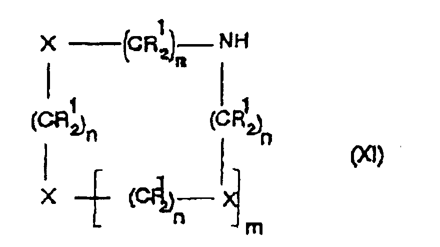 (dans lesquelles X, R¹, n, q et m sont tels que définis dans la revendication 1), ou d'un dérivé activé de ceux-ci, avec une molécule liante de formule IX
Lv(CO-X² )̵L¹-(X²-CO) ₜLv (IX)
ou de formule IXa
Lv-(CR¹ ₂)_{q}-(CO-X² )̵L¹-(X²-CO)ₜ-(CR¹ ₂)_{q}-Lv (IXa)
(dans lesquelles R¹, q, X² et L¹ sont tels que définis dans la revendication 1, t est 0 ou 1, et chaque Lv est un groupe partant déplaçable, l'un étant éventuellement protégé avant la conjugaison du second composé de formule X ou XI) ;
(c) la conversion d'un composé de formule X en un chlorure d'acide correspondant et la réaction avec une diamine appropriée ;
(d) la métallation ou la transmétallation d'un composé de formule V ou d'un chélate de celui-ci ;
(e) la conversion d'un composé de formule V ou d'un chélate de celui-ci en un sel d'addition de celui-ci avec une base ou un acide ou la conversion d'un sel en l'acide ou la base libre ; et
(f) la réalisation d'au moins l'une des étapes (a) à (d) ci-dessus en utilisant des réactifs ayant des groupes fonctionnels protégés puis en éliminant les groupes protecteurs.

13. Procédé pour la préparation d'un chélate métallique, ledit procédé comprenant le mélange dans un solvant d'un composé de formule V tel que défini dans la revendication 1 ou d'un sel ou chélate de celui-ci, avec un composé au moins peu soluble dudit métal.
